(19) Europäisches Patentamt
European Patent Office
Office européen des brevets

(11) Veröffentlichungsnummer: **0 003 274**
**B1**

(12) # EUROPÄISCHE PATENTSCHRIFT

(45) Veröffentlichungstag der Patentschrift: **29.02.84**

(51) Int. Cl.³: **C 07 D 311/60, A 61 K 31/35**

(21) Anmeldenummer: **78810024.6**

(22) Anmeldetag: **22.11.78**

(54) **o-Substituierte Derivate des (+)-Cyanidin-3-ols, Verfahren zu ihrer Herstellung, sowie diese Verbindungen enthaltende pharmazeutische Zusammensetzungen und deren Herstellung.**

(30) Priorität: **25.11.77 CH 14479/77**
**17.03.78 CH 2937/78**

(43) Veröffentlichungstag der Anmeldung:
**08.08.79 Patentblatt 79/16**

(45) Bekanntmachung des Hinweises auf die Patenterteilung:
**29.02.84 Patentblatt 84/9**

(84) Benannte Vertragsstaaten:
**BE CH DE FR GB LU NL SE**

(56) Entgegenhaltungen:
**BE - A - 742 073**
**DE - A - 2 109 602**
**DE - A - 2 206 570**
**DE - A - 2 711 927**
**DE - A - 2 740 346**

**Die Akte enthält technische Angaben, die nach dem Eingang der Anmeldung eingereicht wurden und die nicht in dieser Patentschrift enthalten sind.**

(73) Patentinhaber: **Zyma SA**
**Route de l'Etraz**
**CH-1260 Nyon (CH)**

(72) Erfinder: **Albert, Alban, Dr.**
**62, Av. de Gennecy**
**CH-1249 Avully (CH)**
Erfinder: **Courbat, Pierre, Dr.**
**20, Route de Divonne**
**CH-1260 Nyon (CH)**
Erfinder: **Weith, André, Dr.**
**Résidence les Pins 13**
**CH-1197 Prangins (CH)**

(74) Vertreter: **Meyer, Hans Rudolf et al,**
**Patentabteilung der CIBA-GEIGY AG Postfach**
**CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 274 B1

O-Substituierte Derivate des (+)-Cyanidan-3-ols, Verfahren zu ihrer Herstellung, sowie diese Verbindungen enthaltende pharmazeutische Zusammensetzungen und deren Herstellung

Die Erfindung betrifft neue O-substituierter Derivate des (+)-Cyanidan-3-ols, der Formel

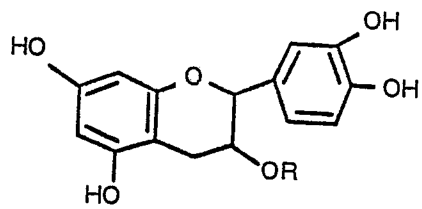

(I)

worin R

einen unsubstituierten oder durch Hydroxy, Niederalkanoyloxy, Niederalkyloxy, Niederalkanoyl, Cyano, Amino, Mono- oder Diniederalkyl-amino, Niederalkylenamino, Oxa- oder Azaniederalkylenamino, Carbamoyl, Mono- oder Diniederalkyl-carbamoyl, oder Halogenatome substituierten Alkyl- oder Alkenyl-Rest,

einen unsubstituierten oder durch eines oder mehrere Hydroxy-, Niederalkoxy, Niederalkyl, Carboxy, Oxo, Niederalkanoyl, Amino, Mono- oder Di-alkyl-amino oder Carboniederalkoxy substituierten Cycloalkyl-Rest mit 3 bis 6 Ringkohlenstoffatomen,

ein Cycloalkyl-niederalkyl-Rest, der im Ring 3 bis 6 Kohlenstoffatome aufweist und unsubstituiert oder durch mindestens einen Niederalkylrest substituiert sein kann,

ein Phenyl- oder Naphthyl-Rest, der durch Hydroxy, Niederalkoxy, Carboxy, Carboniederalkoxy, Nitro, Amino-, Mono-oder Diniederalkylamino, oder Halogenatome, Mono-, di- oder trisubstituiert sein kann,

ein Phenyl- oder Naphtyl-niederalkyl- oder -nieder-alkenyl-Rest der im aromatischen Kern durch Hydroxy, Niederalkoxy, Carboxy, Carboniederalkoxy, Alkanoyl, Nitro, Amino, Mono- oder Dialkyl-amino, Di-niederalkyl-carbamoyl oder Halogenatome, mono- di- oder trisubstituiert sein kann (ausgenommen Trihydroxy),

ein gesättigter oder ungesättigter Aza-, Thia-, Oxa-, Thiaza-, Oxaza- oder Diaza-cyclischer Rest, der an einen Phenylkern ankondensiert sein kann, und der 2—7 Kohlenstoffatome im heterocyclischen Ring aufweist und durch Alkyl, Hydroxy, Niederalkoxy, Nitro, Carboxy, Carbo-niederalkoxy oder Alkanoyloxy mono-, di- oder polysubstituiert sein kann,

ein Heterocyclyl-niederalkyl-Rest, dessen Heterocyclus ein gesättigter oder ungesättigter Aza-, Thia-, Oxa-, Thiaza-, Oxaza- oder Diaza-cyclus darstellt,

ein Rest einer Alkancarbonsäure mit mindestens 3 Kohlenstoffatomen, Niederalkencarbonsäure, Niederalkandicarbonsäure, oder Niederalkendicarbonsäure,

ein unsubstituierter oder durch eine oder mehrere Hydroxy, Niederalkyloxy, Carboxy, Carboniederalkoxy, Niederalkyl, Niederalkanoyl, Oxo oder Halogen substituierter Cycloalkancarbonsäure-Rest,

Benzoyl oder Naphthoylrest, der gegebenenfalls durch ein Hydroxy, Niederalkoxy, Alkanoyloxy, Carboxy, Carboniederalkoxy, Nitro, Amino oder Halogen substituiert sein kann,

ein Phenyl- oder Naphthyl-niederalkanoyl-Rest, der im aromatischen Teil unsubstituiert oder durch Hydroxy, Niederalkoxy, Carboxy, Carboniederalkoxy, Niederalkanoyl, Niederalkanoyloxy, Nitro, Amino, Mono oder Diniederalkylamino oder Halogen substituiert sein kann,

ein Rest einer Aza-, Thia-, Oxa-, Thiaza-, Oxaza oder Diaza-cyclischen Säure, die gesättigt oder ungesättigt, oder auch an einen Phenylrest ankondensiert sein kann,

ein Alkoxycarbonyl-Rest,

ein Phenyloxycarbonyl-Rest,

ein Carbamoyl-Rest, der gegebenenfalls mono- oder disubstituiert sein kann durch Niederalkylreste, Phenyl- oder Benzylreste, die gegebenenfalls im Ring durch Hydroxy, Niederalkoxy, Niederalkanoyloxy, oder Halogenatome substituiert sein können,

ein Rest einer Niederalkyl-sulfonsäure, oder Phenylsulfonsäure, die im Ring durch Niederalkyl, Niederalkoxy oder Halogenatome substituiert sein kann, oder

den Rest einer unsubstituierten oder durch 1 oder 2 Niederalkyl- oder 1 oder 2 Phenylreste veresterten Phosphorsäure sein können, wobei Nieder einen Rest mit bis 7 C-Atomen bedeutet, und ihre Salze, ein Verfahren zu deren Herstellung, sowie diese Verbindungen enthaltende pharmazeutische Zusammensetzungen.

Ein gegebenenfalls substituierter Kohlenwasserstoffrest R kann 1—24 Kohlenstoffatome enthalten. Ein Rest Acyl einer Carbonsäure kann 3—24 Kohlenstoffe enthalten.

Die im Zusammenhang mit der vorliegenden Beschreibung mit "nieder" bezeichneten Reste und Verbindungen enthalten vorzugsweise bis 4 Kohlenstoffatome.

Vorstehend, wie nachfolgend, können die Allgemeinbegriffe folgende Bedeutung haben:

Ein Alkylrest ist vorzugsweise ein Niederalkylrest, wie z.B. Methyl, Aethyl, n-Propyl, Isopropyl, n-Butyl, Isobutyl, Butyl, sec. oder tert. Butyl, aber auch Pentyl, Isopentyl, n-Hexyl, Isohexyl, n-Heptyl, Octyl, Nonyl, Decyl, Indecyl, Dodecyl, Tricosyl, Tetracosyl und deren Isomere. Ein Alkenylrest ist vorzugsweise ein Niederalkenyl, z.B. Vinyl, Allyl, n-Propenyl, Isopropenyl, 2- oder 3-Methylallyl oder 3-Butenyl. Ein Alkynylrest ist z.B. der Propargyl oder 2-Butyrylrest.

Die Acylreste von Alkancarbonsäuren sind mit mindestens 3 Kohlenstoffatomen besonders solche der Propionsäure, Buttersäure, Isobuttersäure, Valeriansäure und deren höhere Homologen bis zur Stearinsäure, die der Alkandicarbonsäuren besitzen, z.B. 2—10, vorzugsweise 3—6 Kohlenstoffatome und die Niederalkendicarbonsäuren, z.B. 4—7 Kohlenstoffatome. Als solche sollen genannt werden: 2-Methyl-bernsteinsäure, Glutarsäure, 3-Methyl-glutarsäure, 3-Aethylglutarsäure, Adipinsäuren, Pimelinsäure, Suberansäure, Azealinsäure, oder Sebazinsäure, vorzugsweise die Malonsäure und Bernsteinsäure. Als Acylreste von ungesättigten aliphatischen Säuren sind besonders diejenigen von Acrylsäuren, Propionyl-, Methacryl-, Croton- oder Oelsäure, in erster Linie von Maleinsäure oder Fumarsäure zu nennen.

Die Acylreste von carbocyclischen Säuren sind in erster Linie solche von Cyclohexan-carbonsäure und Benzoesäuren, die z.B. durch Niederalkyl, wie Methyl, Alkoxyl, wie Methoxy oder Aethoxy oder eine Carboxygruppe substituiert sein können; unter diesen sind besonders Phthalsäure, Isophthalsäure, Terephthalsäure, Zimtsäure oder Toluolsäure, wie auch 1- oder 2-Naphtoesäure oder 1,2-Cyclohexandicarbonsäure zu nennen.

Die Acylreste von heterocyclischen Säuren sind z.B. solche der Pyrancarbonsäure, Thienylcarbonsäure, Nikotin-, Isonikotin- oder Picolin-säure.

Eine Niederalkoxycarbonylgruppe ist besonders Methoxycarbonyl, Aethoxycarbonyl, n-Propyloxycarbonyl, Isopropyloxycarbonyl, Tertiobutyloxycarbonyl oder Tertiopentyloxycarbonyl.

Gegebenenfalls N-substituierte Carbamoylgruppen sind z.B. N-niederalkyl oder N,N-diniederalkylcarbomoyl wie N-methyl-, N-äthyl-, N,N-dimethyl-, oder N,N-diäthylcarbamoyl, N-aryl-, N,N-diaryl- oder N-aryl-N-alkyl-carbamoyl, wie N-phenyl-, N,N-diphenyl- oder N-phenyl-N-methyloder äthyl-carbamoyl die im Phenylkern unsubstituiert oder substituiert sein können.

Eine Niederalkoxygruppe ist z.B. Methoxy, Aethoxy, n-Propyloxy, Isopropyloxy, n-Butyloxy, Isobutyloxy, sec.-, oder tert.-butyloxy, n-pentyloxy, oder tert.-pentyloxy.

Ein Halogen ist z.B. Brom, Jod und besonders Chlor oder Fluor.

Eine mono- oder Diniederalkylgruppe ist z.B. Methylamino, Dimethylamino, Aethylamino oder Diäthylamino.

Eine Niederalkylenaminogruppe, die auch durch Heteroatome unterbrochen sein kann, ist z.B. Pyrrolidino, Piperidiono, Morpholino, Thiamorpholino oder 4-Methyl-piperazino.

Die neuen Verbindungen, die einen salzbildenden Rest aufweisen, können auch in Form ihrer Salze vorliegen.

Als Salze von Verbindungen mit einer freien Carbonsäure, sollen z.B. Metallsalze, besonders solche mit Alkalimetallen wie Natrium oder Kalium, oder auch mit Erdalkalimetallen wie Magnesium oder Kalzium oder z.B. mit Zink, Kupfer, Eisen, Silber oder Quecksilber oder mit Ammonium z.B. Ammoniak oder organischen Basen wie Niederalkylaminen, z.B. Trimethylamine oder Triäthylamin, im besondern nicht toxische und pharmazeutisch verwendbare vom obigen Typus genannt werden.

Die neuen Verbindungen welche basische Gruppen enthalten können Säureadditionssalze bilden, besondern mit nicht toxischen pharmazeutisch verwendbaren Säuren, wie Mineralsäuren z.B. Chlorwasserstoff- oder Bromwasserstoffsäuren, Schwefelsäuren, Phosphorsäure, Carbonsäuren oder organischen Sulfonsäuren z.B. aliphatischen, cycloaliphatischen, cycloaliphatisch-aliphatischen, aromatischen, araliphatischen, heterocyclischen oder heterocyclisch-aliphatischen Säuren z.B. Essigsäure, Propionsäure, Bernsteinsäure, Glycolsäure, Milchsäure, Maleinsäure, Zitronensäure, Weinsäure, Ascorbinsäure, Maleinsäure, Phenylessigsäure, Benzylsäure, 4-Aminobenzylsäure, Anthranilsäure, 4-Hydroxybenzoesäure, Salicylsäure, Aminosalicylsäure, Embonsäure oder Nikotinsäure, Methansulfonsäure, Aethansulfonsäure, 2-Hydroxyäthansulfonsäure, Phenylsulfonsäure, 4-Methyl-phenylsulfonsäure, Naphthalinsulfonsäure, Sulfanilsäure oder Cyclohexylsulfanylsäure.

Die neuen Verbindungen welche basische Gruppen enthalten können auch in Form ihrer quaternären Ammoniumverbindungen vorliegen.

Die neuen Verbindungen besitzen wertvolle pharmakologische Eigenschaften. Insbesondere haben sie eine interessante Wirk-samkeit bei der Verhinderung der hepatischen Nekrose und verhindern die Lipoperoxidation. Sie können auch den Abbau des Kollagens verhindern, wie auch die Wirksamkeit der lysosomialen Enzyme durch Erhöhung der Stabilität der lysosomen Membranen. Sie können auch die Permeabilität und den Gefässtonus beeinflussen.

Die neuen Verbindungen eignen sich besonders für die Behandlung von Leberkrankheiten, wie der akuten Hepatitis (viral, alkoholisch oder toxisch), die Steatosen und die chronische Hepatitis, insbesondere infolge von Alkoholmissbrauch. Sie können auch verwendet werden zur Behandlung von Krankheiten welche eine Veränderung des Konjunktivgewebes, besonders angeborener krankheiten des Kollagens, wie die Ehlers-Danlos-Krankheit, das Marfan-Syndrom, die Cutix-Laxa, aber auch die Skoliose, Osteoporose, Sclerodermie, Paradontose, zur Heilung von Wunden, besonders bei Geschwuren des Decubitus, wie auch bei bestimmten Formen des Rheumatismus im besonderen

der Arthrose. Sie können auch bei Zirkulationsstörungen venöser oder arterieller Art verwendet werden.

So kann z.B. die Wirksamkeit auf den normalen oder pathologischen Metabolismus von Hepatozyten von Ratten die nach der Technik von Berry und Friend [J. Cell. Biol. *43,* 506—520 (1969)] isoliert wurden und in 2 ml einer physiologischen Lösung von Krebs-Ringer in Gegenwart von 0,5 bis 5 mg der neuen Produkte inkubiert wurden und denen man verschiedene hepatotoxische Substanzen zufügt, oder durch die Verhinderung der Lipoperoxidation durch Tetrachlorkohlenstoff, gemäss der Methode von Comporti, Sacconi und Danzani [Enzymologia *28,* 185—203 (1965)], wobei die Intensität der Lipoperoxidation in Gegenwart von 5 bis 50 $\mu$g pro 4 ml durch die Menge des gebildeten Malondialdehyds evaluiert wird. Die Veränderung der experimentellen Hepatitis, eingeleitet durch Galaktosaminen, Tetrachlorkohlenstoff oder Aethanol kann an Ratten gezeigt werden, welche die neuen Produkte oral oder intraperitoneal in Dosen von 100—500 mg/kg erhielten. Bei den akuten Statien werden die Tiere nach 24 oder 4 Stunden nach der Gabe des toxischen Mittels getötet und der Leberzustand mit den folgenden Tests beurteilt: clearance in BSP, die Menge von Bilirubin im Plasma, die Menge Transaminase im Plasma, die Menge von Triglycerin und Lipinen in der Leber und im Serum, der Serumlipoproteine. Damit wird gezeigt, dass die neuen Substanzen fähig sind, den Leberregenerationsprozess günstig zu beeinflussen und einen immunostimulierenden Effekt auszulösen.

Die Wirkstamkeit der neuen Verbindung zum Schutze des Konjunktivgewebes kann gezeigt werden durch die Verhinderung des Abbaus des Kollagens durch die Kollagenase mit 0,1 bis 5 mg/2 ml de neuen Substanzen oder durch die Verhinderung der Wirksamkeit der lysomialen Enzyme und die Erhöhung der Stabilität der lysosomen Membrane mittels 0,05 bis 0,2 mg/l ml der neuen Verbindung gemäss P. Niebes und Ponard, [biochem. Pharmacol. *24,* 905 (1975)] oder auch durch den experimentellen Lathyrismus bei Ratten denen man 100 mg/kg/Tag Aminoacetonitril p.o. oder 250 mg/kg/Tag Iminodipropionitril subcutan während 7 Tagen oder 600 mg/kg/Tag $\beta$-Aminopropionitril oral während 3 Wochen und denen man 100—500 mg/kg der neuen Produkte oral während 3 Wochen vor, während oder nach der Vergiftung gibt. Bei Dosen von 100—500 mg/kg parenteral oder oral gegeben, können diese neuen Substanzen bei diesen Tieren a) das experimentelle Oedem, das durch Galaktosamin und Dextran hervorgerufen wird, vermindern, b) die kapillare Permeabilität bei der Ratte gemessen durch die Diffusion von Tripan-Blau [V. L. Beach und B. G. Steinetz, J. pharmacol. Exp. Ther. *131*, 400 (1961)], vermindern, c) den Durchfluss venal retro-aurikular am Kaninchenohr vermindern, d) die Reinigung des ischemischen Pfote des Hundes von Gewebsmilchsäure zu begüngstigen.

Die Wirksamkeit dieser neuen Verbindungen konnte bis jetzt nicht beobachtet werden bei diesen Indikationen. Bis zur Stunde wusste man dass das (+)-Cyanidan-3-ol einen Leberschutzeffekt aufweist. Es ist überraschend, dass die neuen Verbindungen zusätzliche und höhere Wirksamkeiten als diejenige des (+)-Cyanidan-3-ols ausweisen.

Die Erfindung betrifft besonders die in den Beispielen beschriebenen Verbindungen der Formel I.

Der Grundstoff, das (+)-Cyanidan-3-ol, und einige Derivate waren aus der Literatur (s. Liebigs Anm. Chem. *714,* 193—204 (1968); DE—A 2 711 927; DE—A 2 206 570; BE—A 742 074; DE—A 2 740 346 und DE—A 2 109 602). Ein Wirkungsvergleich von erfindungsgemäss erhältlichen Verbindungen (3-O-Butyryl-(+)-cyandian-3-ol) mit einer Verbindung gemäss dem Stand der Technik (Penta-O-butyryl-3,5,7,3',4'-(+)-cyanidan-3-ol, gemäss DE—A 2 711 927) zeigte bei der durch Galactosamin an der Rate ausgelösten Hepatitis (wie oben beschrieben) eine überraschende Ueberlegenheit der erfindungsgemäss erhältlichen Verbindung.

Die neuen Verbindungen können in an sich bekannter Weise erhalten werden. So kann man sie z.B. erhalten wenn man in einer Verbindung der Formel II

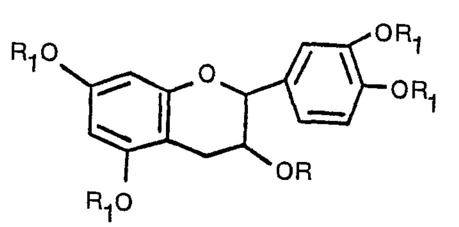

(II)

worin $R_1O$ eine geschütze Oxygruppe, die leicht hydrolisierbar oder hydrogenolysierbar ist, darstellt und R die obgenannte Bedeutung besitzt, den Rest $OR_1$ zur Hydroxygruppe aufspaltet und wenn erwünscht in einer erhaltenen Verbindung einen Rest R durch Reduktion umwandelt, und/oder, dass man ein erhaltenes Salz in die freie Verbindung oder eine freie Verbindung mit salzbildenden Gruppen in ein Salz umwandelt.

In den Verbindungen der Formel II kann der Rest $R_1$ ein Neideralkylrest, der mono-, die- oder polysubstituiert ist, ein Niederalkenylrest, einen unsubstituierten oder mono-, di- oder trisubstituierten araliphatischen Rest, einen aliphatischen, aromatischen oder araliphatischen Carbonsäurerest oder ein veresterter Kohlensäurerest, oder ein Rest der mit dem an $R_1$ gebundenen Sauerstoffatom ein cyclisches Acetal bildet.

Wenn $R_1$ ein Niederalkylrest darstellt enthält dieser vorzugsweise 1—4 Kohlenstoffatome und ist in erster Linie Methyl oder Aethyl. Diese Reste können durch Niederalkoxy, z.B. Aethoxy oder Methoxy, oder Niederalkoxy-aethoxy, z.B. Methoxyäthoxy substituiert sein.

Ist $R_1$ ein Niederalkylenylrest, enthält dieser vorzugsweise 2—5 Kohlenstoffatome und ist in erster Linie Allyl, Methallyl, 2-Butenyl oder 2-Pentenyl.

Ist $R_1$ ein araliphatischer, insbesondere ein Benzylrest, kann dieser verschieden substituiert sein, z.B. durch 1 oder mehrere Alkyl mit 1—4 Kohlenstoffatomen, wie Methyl, Aethyl, Isopropyl oder Tert.-Propyl, durch ein oder mehrere Halogene vorzugsweise Chlor, Brom oder Fluor, ein oder mehrere Alkoxy, wie Methoxy oder Aethoxy oder auch durch Nitril. Diese Substituenten können die ortho- und para-Stellung besetzen; bevorzugt sind sie in para-Stellung. Ist ein Substituent im Rest $R_1$ von zweiter Ordnung wie die Nitrogruppe, sitzt er vorzugsweise in meta-Stellung.

Ist der Rest $R_1$ ein aliphatischer Acylrest, ist der aliphatische Teil z.B. ein Alkyl mit 1—4 Kohlenstoffatomen und vorzugsweise Methyl oder Aethyl, die durch Niederalkoxy, wie Methoxy substituiert sein können. Ist $R_1$ ein aromatischer oder araliphatischer Acylrest, ist der aromatische Rest besonders ein Phenylrest der gegebenenfalls substituiert ist, und der aliphatische Rest ein Alkyl mit 1—2 Kohlenstoffatomen.

Ist $R_1$ ein Alkoxycarbonyl, kann der Alkylrest wiederum durch ein Aryl substituiert sein oder durch ein Halogenatom. Ist $R_1$ ein Acetal zusammen mit dem Sauerstoff das den Rest $R_1$ trägt, kann dieses Acetal linear oder cyclische sein, beispielsweise, ein Pyranylrest.

Unter den Resten der Formel $R_1$ sollen besonders genannt werden, z.B. Allyl, Methallyl, Aethylallyl, 2-Butenyl, 2-Pentenyl, Methoxymethyl, Aethoxymethyl, Methoxyäthoxymethyl, Acetonyl, Propionylmethyl, Cinnamyl, Phenacyl, Benzyl, 2-Methylbenzyl, 4-Methylbenzyl, 4-t-Butyl-benzyl, 4-Brombenzyl, 4-Fluorbenzyl, 4-Chlorbenzyl, 4-Methoxybenzyl, 4-Aethoxybenzyl, 3-Nitrobenzyl, 3,5-Dinitrobenzyl, 4-Cyanbenzyl, Chloro-, Bromo- oder Phenyl-phenacyl oder Methoxycarbonyl, Aethoxycarbonyl, 2,2,2-Trichloräthoxycarbonyl, oder Phenoxycarbonyl.

Die $OR_1$-Gruppen können durch Hydrolyse oder durch Hydrogenolye gespaltet werden.

Die Hydrolyse dieser Substanzen kann mit Säuren erfolgen, jedoch besteht hierbei die Gefahr, dass sich die Flavon-Struktur verändert und entsprechend die Ausbeute sinkt. Vorzugsweise verwendet man p-Toluolsulfonsäure, wobei man die Reaktion vorzugsweise in einem Alkohol wie Methanol durchführt. Je nach der Art der Substituenten kann die Reaktion eine oder mehrere Dutzende Stunden dauern. Sie kann bei Raumtemperatur durchgeführt werden oder auch durch Kochen am Rückfluss beschleunigt werden, wobei das Risiko einer teilweisen Zersetzung der Endprodukte in Kauf genommen werden muss.

Man kann die Gruppe $OR_1$ auch durch katalytische Hydrierung mit molekularem Wasserstoff oder aber auch durch Wasserstofftransfer in situ mittels eines Wasserstoffgebers wie Cyclohexen durchführen. Falls die zu hydrierende Verbindung einen salzbildenden Rest im Substituenten $R_1$ besitzt, kann man die freie Form oder ein Salz davon verwenden.

Die Reaktion wird in Gegenwart eines Lösungsmittels, wie Wasser, Methanol, Aethanol, Isopropanol, tert.-Butylalkohol, Aethylacetat, Dioxan oder Tetrahydrofuran durchgeführt. Sie verläuft bei Raumtemperatur unter Normaldruck, man kann aber auch bei höherer Temperatur und/oder höherem Druck arbeiten. Vorzugsweise wählt man solche Reaktionsbedingungen und Katalysatoren, dass eine Zerstörung des Flavanylrestes vermieden und andererseits weder der aromatische Kern noch die gewünschten Substituenten R zerstört werden. Als Katalysatoren sind besonders fein verteiltes Palladium oder Palladium auf Tierkohle zu nennen. Man arbeitet dabei vorzugsweise bei Raumtemperatur und Normaldruck.

Besitzen die Reste R hydrierbare Gruppen, können sie gleichzeitig hydriert werden, besonders wenn man stark reaktive Katalysatoren wie Palladium auf Kohle verwendet. Es ist auch möglich, bei Verminderung der Katalysatoraktivität die Gruppe R intakt zu belassen.

Die Verbindungen welche eine salzbildende Gruppe enthalten, wie beispielsweise Carboxylgruppen können je nach den verwendeten Bedingungen in freier Form oder in Form ihrer Salze erhalten werden. Diese Formen können gegenseitig Eine in die Andere übergeführt werden. Salze von Verbindungen die eine freie Carbonsäure enthalten, sind z.B. Metallsalze, im besondern Alkalimetallsalze z.B. Natrium oder Kaliumsalze, wie auch Erdalkalimetallsalze, z.B. Magnesium- oder Kalziumsalze, oder Ammoniumsalze z.B. mit Ammoniak oder organischen Basen, wie Triniederalkylamin, z.B. Trimethylamin oder Triäthylamin. Man erhält diese Salze z.B. indem man die freie Verbindung mit Hydroxyden oder Carbonaten dieser Metalle oder mit Ammoniak oder Aminen, oder auch mit geeigneten Ionenaustauschern, oder mit metallorganischen Verbindungen umsetzt.

Verbindungen die basische Gruppen aufweisen, können sich auch in Form ihrer Säureadditionssalze, besonders mit nicht giftigen pharmazeutisch verwendbaren Säuren, z.B. mit Mineralsäuren, wie Salzsäure, Bromwasserstoffsäure, Schwefelsäure oder Phosphorsäure, oder mit organischen Carbon oder Sulfonsäuren z.B. aliphatischen, cycloaliphatischen, cycloaliphatischen-aliphatisch, aromatischen, araliphatischen, heterocyclischen oder heterocyclisch-aliphatischen Säuren, z.B. Essigsäure, Propionsäure, Bernsteinsäure, Glyconsäure, Milchsäure, Maleinsäure, Weinsäure, Zitronensäure, Ascorbinsäure, Phenylessigsäure, Benzoesäure, 4-Aminobenzoesäure, Anthranilsäure, 4-Hydroxybenzoesäure, Salicylsäure, Aminosalicylsäure, Embonsäure oder Nikotinsäure, wie auch Methansulfonsäure, Aethansulfonsäure, 2-Hydroxyäthan-sulfonsäure, Aethylensulfonsäure, Phenylsulfonsäure, p-Methylphenylsulfonsäure, Naphthalinsulfonsäure, Sulfanylsäure, oder Cyclohexylsulfaminsäure. Diese Salze können z.B. durch Behandlung der freien Verbindungen die eine basische Gruppe enthalten mit den gennanten Säuren oder entsprechenden Anion-Austauschern behandelt werden.

Die neuen Verbindungen enthaltend basische Gruppen, können auch in ihre quaternäre Ammoniumsalze z.B. durch Behandlung mit Alkylierungsmitteln wie Niederalkylhalogeniden z.B. Jodmethyl, Niederalkyl- oder Benzylsulfaten wie Methylsulfat erhalten werden.

Die Ausgangsstoffe zu diesem Verfahren sind zum Teil bekannt, zum grössern Teil jedoch neu. Sie können nach an sich bekannter Methode oder aber besonders durch eine neue und vorteilhafte Weise gewonnen werden. So kann man z.B. (+)-Cyanidan-3-yl oder eines seiner Salze mit einem reaktionsfähigen Ester eines Alkohols der Formel

$$HO—R_1$$

worin $R_1$ die obengenannte Bedeutung hat, umsetzen, und die Reste R in die Hydroxygruppe in 3-Stellung durch Verätherung oder Veresterung einführen.

Eine reaktionsfähige Ester ist besonders ein Ester mit einer starken anorganischen Säure, in erster Linie mit einer Halogenwasserstoffsäure, wie Chlor- oder Bromwasserstoff oder einer oganischen Sulfonsäure, vorzugsweise einer Niederalkansulfonsäure z.B. Methan- oder Aethansulfonsäure oder einer Benzolsulfonsäure die im Benzolkern z.B. durch Methyl, Chlor oder Brom substituiert sein kann, wie die p-Toluolsulfonsäure oder p-Brombenzosulfonsäure.

Durch dieses Verfahren erhält man Derivate des (+)-cyanidan-3-ols deren 4-phenolische Hydroxygruppen substituiert sind. Unter diesen Tetraäthern wurde das 5,7,3',4'-tetra-O-Benzyl-(+)-cyanidan-3-ol bereits durch ein reines Laboratoriumsverfahren hergestellt. [K. Weinges et D. Seiler, Liebigs Ann. Chem. *714* 193—204 (1968)]. Dabei wurde (+)-Cyanidan-3-ol gelöst in Aceton mit Benzylchlorid in Gegenwart von Kaliumjodid und Kaliumcarbonat unter Stickstoff und mehrstündigem Kochen am Rückfluss umgesetzt. Es handelt sich dabei um eine klassische Benzylierung und sie führt nach Trennung und Kristallisationen aus Aethanol zu einer Mischung von 8-Benzyl-5,7,3',4'-tetra-O-Benzyl-(+)-cyanidan-3-ol und 5,7,3',4'-tetra-O-benzyl-(+)-cyanidan-3-ol. Das Letztere wird durch Chromatographie mit einer Ausbeute von 1—2% isoliert. Diese Ausbeute ist so klein, dass diese Methode zur Herstellung der tetrasubstituierten Verbindungen industriell nicht verwendet werden kann.

Ersetzt man jedoch das Benzylchlorid durch ein anderes Halogenid, ausgewählt aus den Resten $R_1$, kann sie gut durchgeführt werden, besonders wenn man Chloride oder Bromide von un- oder substituiertem Phenacyl, z.B. Phenacyl-chlorid oder -bromid, oder 4-Bromphenacyl oder 4-Phenylphenacyl-chlorid oder -bromid verwendet. Nach dem Abfiltrieren des unlöslichen Kaliumcarbonats und dem Eindampfen des Acetons reicht es, den Rückstand in einem geeigneten Lösungsmittel z.B. einer Mischung von Aceton und Methanol zu kristallisieren. Man erhält so die Tetraäther mit guter Ausbeute.

Es wurde ausserdem gefunden, dass man die Zwischenprodukte mit sehr guter Ausbeute erhalten kann, wenn man (+)-Cyanidan-3-ol in einem aprotischen und organischen Lösungsmittel mit hoher Dielektrizitätskonstante mit einem Alkalihydrid oder Alkalicarbonat behandelt und das erhaltene Tetraalkalisalz mit einem reaktionsfähigen Ester der Formel

$$HO—R_1$$

umsetzt, wobei das Verhältnis Cyanidan-3-ol, Alkalihydrid und reaktionsfähiger Ester 1: ungefähr 4,25: ungefähr 4,5 beträgt oder das Verhältnis Cyanidan-3-ol, Alkalicarbonat und reaktionsfähiger Ester 1: ungefähr 8: ungefähr 6 beträgt.

Das Alkalihydrid ist besonders Natriumhydrid und wird vorzugsweise in Form einer Dispersion in Oel verwendet. Das Alkalicarbonat ist besonders Kaliumcarbonat.

Die verwendeten aprotischen Lösungsmittel sind besonders Amide, wie Dimethylformamid, aber auch Sulfoxyde, wie Dimethylsulfoxyde. Das Dimethylformamid wird jedoch aus ökonomischen Gründen vorgezogen, weil man es durch einfache Destillation bei Normaldruck wieder erhalten kann, und weil es einfacher zu handhaben ist als Dimethylsulfoxyd.

Im übrigen ist es wichtig, dass das Reaktionsmilieu so trocken als nur möglich ist. So trocknet man das Dimethylformamid über einem Molekularsieb und trocknet auf gleiche Weise die Lösung von Cyanidanol in Dimethylformamid. Die Bildung von Nebenprodukten wird dabei vermieden, ohne dass das Trocknen der Bildung der gewünschten Produkte entgegenstehe.

Die Reaktion kann in Gegenwart eines quaternären Ammoniumsalzes wie Tetra-n-butylammoniumhydrogensulfat, das in einer Proportion von 0,1 bis 0,5 Mol per 1 Mol Cyanidan-ol verwendet werden, oder eines Crown-äthers, vorzugsweise 18-Crown-6 der in einer Menge von 0,5 Mol per 1 Mol Cyanidan-ol verwendet wird, durchgeführt werden.

Man führt die Reaktion bei einer Temperatur zwischen —25°C und ungefähr +50°C durch. Da es bekannt ist, dass das Dimethylformamid sich bereits bei Raumtemperatur im Kontakt mit basischen Substanzen zu zersetzen beginnt, führt man diese Reaktion vorzugsweise bei —25°C bis +25°C und in erster Linie bei ungefähr —5°C bis ungefähr 0°C durch. Mit Dimethylsulfoxyd, da es sich kaum zersetzt,

kann man die Reaktion bei einer Temperatur die niederer ist als 50°C, vorzugsweise zwischen +15°C und ungefähr +30°C z.B. bei Raumtemperatur durchführen.

Da das Alkalisalz des (+)-Cyanidan-3-ols in Dimethylformamid-natriumhydrid selbst bei niederer Temperatur nicht stabil ist, muss man das Veresterungs- oder Verätherungsmittel sofort zugeben. Wird das nicht gemacht, wird die Reaktionsausbeute vermindert und die Reinigungsphase des gewünschten Produktes erschwert. Nach der Zugabe des Veresterungs- bzw. Verätherungsmittels bei tiefer Temperatur wird diese noch eine gewisse Zeit beibehalten bevor man langsam die Temperatur ansteigen lässt, z.B. auf Raumtemperatur. Die Reaktion ist dann beendet und die Reaktionsmischung kann mehr als 15 Stunden in diesem Zustand aufbewahrt werden ohne dass sie die Qualität des gewünschten Produktes verändert. Während der Reaktion ist gutes Rühren notwendig.

Das Fortschreiten der Reaktion kann durch Dünnschichtchromatographie auf Silikagel unter Verwendung von Chloroform oder Dichlormethan als mobile Phase, kontrolliert werden.

Nachdem das Lösungsmittel aus dem Reaktionsmedium durch Destillieren entfernt wurde, nimmt man den Rückstand in einem aprotischen Lösungsmittel, wie es zur Kristallisation des Zwischenproduktes verwendet wird, auf. Als Lösungsmittel zur Kristallisation des gewünschten Produktes eignet sich besonders Trichloräthylen. Auch andere Lösungsmittel wie Tetrachlorkohlenstoff, Toluol, Aethylacetat, Aethanol, Isopropanol oder Mischungen dieser Lösungsmittel z.B. mit Tetrachlorkohlenstoff, n-Hexan, oder Aceton-methanol, geben gute Resultate. Aethylenäther und n-Hexan wie auch Aceton, Pyridin, Chloroform, Dimethylformamid, Dimethylsulfoxid, Tetrahydrofuran und Dichlormethan eignen sich weniger gut da die neuen Substanzen darin bei Raumtemperatur löslich sind.

Die Einführung des Restes R in die Zwischenprodukte erfolgt nach an sich bekannten Methoden. So kann man z.B. ein Halogenderivat der Formel

hal—R

auf diese Verbindungen einwirken lassen, vorzugsweise in Gegenwart einer Base, wie Alkali, z.B. Natrium- oder Kaliumhydroxyd, Silberoxyd, Natriumamid, Natrium- oder Kaliumhydrid. Diese Verätherung kann in einem nicht reaktionsfähigen Lösungsmittel wie Dioxan, Tetrahydrofuran, Toluol, gegebenenfalls auch in einer Mischung dieser Lösungsmittel oder einer Mischung mit Wasser durchgeführt werden. Sie wird bei Raumtemperatur vorgenommen und kann durch Wärmen beschleunigt werden. Die Dauer der Reaktion liegt zwischen einigen Minuten und mehreren Tagen, im allgemeinen genügen jedoch einige Stunden. Ist die verwendete Base Soda oder Kaliumcarbonat in wässriger Lösung und das Zwischenprodukt ist in einer Lösung die mit Wasser nicht mischbar ist, kann man sich mit einem Phasentransfer-Katalysator wie Tetra-butyl-ammoniumhydrogensulfat behelfen.

Ist R ein Hydroxyalkyl oder ein Aminoalkyl, kann man auch die Derivate der Formel II durch Kondensation mit Epoxyd oder mit Aziridin in basischem oder saurem Milieu erhalten.

Ist R ein aromatischer Rest kann man die Derivate der Formel II durch Reaktion mit einem aromatischen Halogenid erhalten. Diese Reaktion wird in einem inerten Lösungsmittel wie Tetrahydrofuran bei Raumtemperatur oder höherer Temperatur in Gegenwart einer Base wie tertiären Amin z.B. Trimethyl oder Triäthylamin oder einem Fluorid wie Kaliumfluorid oder einem quaternären Ammoniumsalz in Gegenwart z.B. eines Crown-äthers durchgeführt. Ein Alkoholat der Zwischenprodukte reagiert z.B. mit einem heterocyclischen Halogenid, man kann auch einen aromatischen Diester der Phosphorsäure in Gegenwart von katalytischen Menge einer Sulfonsäure wie der p-Toluol-sulfonsäure verwenden. Die Zwischenprodukte der Formel II in denen R Wasserstoff ist, addieren Olefine die durch elektronenanziehende Gruppen substituiert sind wie Nitril, Nitro, Ketone, oder Ester in Gegenwart einer Base. Die Reaktionsteilnehmer können in einem inerten mit Wasser nicht mischbaren Lösungsmittel gelöst werden und die Base kann dann 50%ige wässrige Soda sein. Man kann auch einen Phasentransfer-Katalysator wie Benzyltrimethylammoniumhydroxyd verwenden. Zu Einführung des Restes R in die Zwischenprodukte verwendet man vorzugsweise ein Derivat einer Säure der Formel

HO—R

wie ein Säurehalogenid, ein Anhydrid oder ein Cyanür die auch in situ gebildet werden können. Man verwendet ein basisches Lösungsmittel wie Pyridin in Ab- oder Anwesenheit eines tertiären aliphatischen Amins wie Triäthylamin und/oder Pyridin oder eine Mischung von Lösungsmittel von denen mindestens eines basisch ist oder ein inertes Lösungsmittel oder ein basisches Lösungsmittel und einen Katalysator vorzugsweise Alkali. Diese Reaktion geht auch bei den N-substituierten Isocyanaten.

Die Ester einer Mineralsäure oder einer Sulfonsäure mit den Zwischenprodukten können durch Reaktion dieser mit einem Halogenid der genannten Säuren oder mit einer Mineralpolysäure die zur Veresterung noch geeignet ist, und bei welcher eine oder zwei Hydroxygruppen substituiert oder blockiert sind. Die Schutzgruppen werden dann durch Hydrolyse oder durch Hydrogenolyse einzeln oder zusammen mit den Resten $R_1$ abgespalten. Die Ester mit Mineralsäuren können auch durch Umsetzung ihrer Anhydride erhalten werden. In Folge der engen Beziehungen zwischen der neuen Verbindungen in freier Form und in Form ihrer Salze sind in vorausgegangenen und nachfolgend unter den freien Verbin-

dungen sinn- und zweckgemäss gegebenenfalls auch die entsprechenden Salze zu verstehen. Die Substituenten R können chiral sein und die neuen Verbindungen sich als reine Stereoisomere oder stereoisomeren Mischungen vorhanden sein. Die Letztern können auf der Basis ihrer physicochemischen Eigenschaften in an sich bekannter Weise in die reinen Stereoisomeren getrennt werden z.B. mit Hilfe einer chromatographischen Trennung wie einer Dünnschichtchromatographie oder irgend einem andern geeigneten Trennungsmittel. Man isoliert vorzugsweise das Aktivere der Stereoisomeren.

Die oben beschrieben Verfahren werden in an sich bekannter Art und Weise, in Gegenwart oder in Abwesenheit von Lösungsmitteln und Verdünnungsmitteln, wenn notwendig unter Kühlen oder Erwärmen, unter erhöhtem Druck und/oder einer inerten Gasatmosphäre wie Stickstoff durchgeführt. Die Erfindung betrifft auch diejenigen Ausführungsformen des Verfahrens, nach dem man von einer auf irgend einer Stufe des Verfahrens als Zwischenprodukt erhältlichen Verbindung ausgeht, und die fehlenden Verfahrensschritte ausführt, oder bei denen man einen Ausgangsstoff unter den Reaktionsbedingungen bildet oder bei denen eine Reaktionskomponente gegebenenfalls in Form ihrer Derivate, wie ihrer Salze und/oder in Form von Isomerengemischen oder reinen Isomeren einsetzt.

Die vorliegende Erfindung betrifft ebenfalls pharmazeutische Präparate, welche Verbindungen der Formel I oder pharmazeutisch verwendbare Salze von solchen Verbindungen mit salzbildenden Gruppen enthalten. Sie enthalten eine wirksame Menge der Aktivsubstanzen zusammen oder in Mischungen mit organischen oder mineralischen Trägersubstanzen die fest oder flüssig sein können und die geeignet sind zur enteralen oder parenteralen Gabe. Vorzugsweise verwendet man Tabletten oder Gelatine-Kapseln enthaltend die aktiven Substanzen zusammen mit einem Verdünnungsmittel wie Lactose, Dextrose, Sukrose, Mannitol, Sorbitol, Cellulose und/oder Glycin, und/oder Schmiermittel wie Silicium, Talk, Stearinsäure oder Salze davon, wie Magnesiumstearat oder Kalziumstearat und/oder Polyäthylenglykol; die Tabletten enthalten auch Bindemittel z.B. Magnesium oder Aluminiumsilicat, Stärken wie Mais, Weizen, Reis oder Kartoffelstärke, Gelatine, Traganth, Methylcellulose, Carboxymethylcellulose und/oder Polyvinylpyrrolidon und wenn erwünscht auch Sprengmittel wie Stärke, Alginsäure, oder Salze davon wie Natriumalginat oder Absorptionsmittel, Farbstoffe, Substanzen die den Geschmack verbessern und/oder Süssmittel. Die Injektionspräparate sind z.B. Lösungen oder Suspensionen in isotonischem Wasser, und die Supositorien sind in erster Linie Fettemulsionen oder Suspensionen. Die pharmazeutischen Präparate können sterilisiert sein und/oder Hilfsstoffe enthalten, wie Konservierungs- und Stabilisierungsmittel und/oder Emulsionsmittel, Salze die den osmotischen Druck regeln und/oder Stabilisatoren. Die gennanten pharmazeutischen Präparate werden in an sich bekannter Weise z.B. mit Hilfe von Granulation und Drageeifizierung erhalten und enthalten zwischen 0,1 bis 75%, im Besondern zwischen 1 und 50% Wirkstoffe.

Die erfindungsgemässen Substanzen werden in einer Dosis von 1 bis 100 mg, vorzugsweise von 1 bis 500 mg per Dosiseinheit verabreicht.

Die nachfolgenden Beispiele illustrieren die oben beschriebene Erfindung; sie sollen jedoch diese in ihrem Umfang in keiner Weise einschränken. Temperaturen werden in Celsius-Graden angegeben.

## Beispiel 1

In einem 3-Liter-Kolben hydriert man eine Suspension von 12 g Palladiumchlorid $PdCl_2$ in 1800 ml Methanol während 2 Stunden bei Raumtemperatur, wobei 2400 ml Wasserstoff verbraucht werden. Man dekantiert das Methanol ab und wäscht das entstandene Palladiumschwarz 4 Mal mit 800 ml Methanol, wobei gebildeter Chlorwasserstoff, sowie Spuren von Palladiumchlorid entfernt werden. Man wäscht dann 3 Mal mit 500 ml Aethylacetat. Beim letzten Dekantieren lässt man eine kleine Schicht Aethylacetat über dem Palladiumschwarz stehen, um eine spontane Entzündung zu verhindern.

Zu dieser Suspension gibt man 40 g 5,7,3',4'-Tetra-O-Benzyl-3-O-methyl-(+)-cyanidan-3-ol in 1600 ml Aethylacetat und hydriert während 3 Stunden, wobei ca. 6600 ml Wasserstoff verbraucht werden. Man filtriert und dampft das Filtrat im Vakuum bei 40° ein. Der Rückstand wird in 2000 ml Wasser aufgenommen und im Vakuum bei 40° auf ein Volumen von ca. 100 ml eingeengt. Diese Reinigung wird 3 Mal wiederholt und am Schluss durch Lyophilisieren getrocknet. Man erhält so 18.5 g 3-O-Methyl-(+)-cyanidan-3-ol vom F = 118—120 $(\alpha)_D^{29}$ = +2,76 (Aethanol, c = 0.5).

## Beispiel 2

In einem 2 liter-Kolben hydriert man 6.0 g Palladiumchlorid in 900 ml Methanol während 1 1/2 Stunden bei Raumtemperatur, wobei ungefähr 1100 ml Wasserstoff verbraucht werden. Man dekantiert das Methanol, wäscht 3 Mal mit 200 ml Methanol, dann 3 Mal mit 200 ml Aethylacetat.

Man fügt eine Lösung von 21,2 g 5,7,3',4'-tetra-O-Benzyl-3-O-butyl-(+)-cyanidan-3-ol in 900 ml Aethylacetat zu und hydriert während 5 Stunden bei Raumtemperatur; ca. 3250 ml Wasserstoff werden verbraucht. Man filtriert vom Palladium ab und dampft das Filtrat im Vakuum bei 40°C ein. Den Rückstand nimmt man in 500 ml Wasser auf und dampft auf ein Volumen von ungefähr 50 ml ein. Dieses wiederholt man 4 Mal. Nach dem letzten Eindampfen trocknet man bei 10 mm Hg und 60° bis zu Gewichtskonstanz. Man erhält so das 3-O-Butyl-(+)-cyanidan-3-ol vom F=103—104°.

## Beispiel 3

Man verfährt wie in Beispiel 2 beschrieben, jedoch ausgehend von 21,6 g 5,7,3',4'-Tetra-O-

benzyl-3-O-butyryl-(+)-cyanidan-3-ol. Nach dem Trocknen, löst man in einer Mischung von 5500 ml Wasser und 1100 ml Aethanol bei Raumtemperatur, filtriert und dampft das Filtrat im Vakuum bei 40° zur Trockne ein. Man dekantiert die Lösung und lässt bei 4° 3 Tage stehen. Der weisse Niederschlag, der sich bildet, wird abfiltriert, mit kaltem Wasser gewaschen und bis zur Gewichtskonstanz bei 10 mm Hg über Phosphorpentoxyd und 50°C getrocknet. Man erhält so das 3-O-Butyryl-(+)-cyanidan-3-ol vom F=112—113°C.

Beispiel 4

Man verfährt wie in Beispiel 2 und erhält aus 22 g 5,3′,4′-Tetra-O-benzyl-3-O-(3,3-dimethyl-butanoyl)-(+)-cyanidan-3-ol das 3-O-(3,3-dimethyl-butanoyl)-(+)-cyanidan-3-ol vom F=118—120°C.

Beispiel 5

Man verfährt wie in Beispiel 2, jedoch ausgehend von 22 g 5,7,3′,4′-tetra-O-Benzyl-3-(3-carboxy-propionyl)-(+)-cyanidan-3-ol. Nach dem Entfernen des Aethylacetats und dem Toluol durch Azeotropismus mit Wasser, löst man den Rückstand in 1000 ml Wasser, dampft bis zum Erscheinen einer leichten Trübung ein (ca 500 ml), filtriert und lyophilisiert das Filtrat bis zur Gewichtskonstanz. Man erhält so das 3-O-(3-carboxy-propionyl)-(+)-cyanidan-3-ol vom F=102—106°C.

Beispiel 6

Man verfährt wie in Beispiel 1, jedoch ausgehend von 50 g 5,7,3′,4′-tetra-O-Benzyl-3-O-decanoyl-(+)-cyanidan-3-ol. Nach dem Abdampfen des Aethylacetats trocknet man den Rückstand im Vakuum bei 40°C, löst ihn in 300 ml Aethanol, engt auf 50 ml ein, gibt 250 ml Aethanol und 250 ml Wasser zu und dampft das Lösungsmittel im Vakuum bei 40°C ab. Der Rückstand wird im Hochvakuum über Phosphorpentoxyd zur Gewichtskonztanz getrocknet. Man erhält so das 3-O-Decanoyl-(+)-cyanidan-3-ol vom F=71—74°C.

Beispiel 7

In einen Zweiliter-Kolben gibt man eine Lösung von 26,6 g 5,7,3′,4′-tetra-O-Benzyl-3-O-palmitoyl-(+)-cyanidan-3-ol in 900 ml Aethylacetat (die Lösung wird durch leichtes Erwärmen erreicht). Dann gibt man 16,0 g Palladium auf Kohle (10%) zu und hydriert das Gemisch. Durch Filtrieren trennt man vom Palladium ab und dampft das Filtrat zur Trockne ein. Der Rückstand, eine klebrige Masse wird in 400 ml heissem Methanol aufgenommen. Unter Rühren fügt man 1500 ml Wasser zu und rührt während einer Nacht weiter bei Raumtemperatur und dann 48 Stunden in einem Eisbad. Der gebildete Niederschlag wird abfiltriert und unter Hochvakuum über Phosphorpentoxyd bei Raumtemperatur getrocknet. Das erhaltene 3-O-Palmitoyl-(+)-cyanidan-3-ol schmilzt bei 67—69°C.

Beispiel 8

Man verfährt wie in Beispiel 2 beschrieben: Das Palladiumschwarz wird durch Hydrierung von 18 g Palladiumchlorid in 900 ml Aethylacetat erhalten, dann hydriert man 40 g 5,7,3′,4′-tetra-Benzyl-3-O-tetrahydrophthalyl-(+)-cyanidan-3-ol in 1800 ml Aethylacetat, wie in Beispiel 1. Nach dem Entfernen der organischen Lösungsmittel durch Azeotropie mit Wasser, trocknet man den Rückstand im hochvakuum und über Phosphorpentoxyd bis zu Gewichtskonstanz. Man erhält so das 3-O-(2-carboxy-cyclohexan-carbonyl)-(+)-cyanidan-3-ol vom F=138—140°C.

Beispiel 9

In einem 10 1 Kolben hydriert man 30 g Palladiumchlorid, das in 4,5 1 Methanol suspendiert wurde. Die Reaktion dauert eine Stunde und verbraucht ungefähr 4.92 1 Wasserstoff. Das erhaltene Palladiumschwarz wird 4 Mal mit je einem Liter Methanol, dann 4 Mal mit je einem Liter Aethylacetat gewaschen. Zum Katalysator, der mit wenig Aethylacetat vermischt ist, fügt man eine Lösung von 113 g 5,7,3′,4′-O-tetra-Benzyl-2-O-benzoyl-(+)-cyanidan-3-ol in 4 Liter Aethylacetat. Man hydriert die Mischung während ungefähr 1 1/2 Stunden, filtriert vom Katalysator ab, wäscht ihn mit 250 ml Aethylacetat, vereinigt die Filtrate und destilliert bei vermindertem Druck bei 40°C. Dur Rückstand wird in 5 Liter Wasser aufgenommen, die erhaltene Suspension 30 Minuten bei Raumtemperatur gerührt, und dampft dann im Vakuum bei 40°C auf ein Volumen von ungefähr 500 ml ein. Man wiederholt dies zwei Mal und trocknet endlich über Silicagel zur Gewichtskonstanz im Hochvakuum. Man erhält so das amorphe 3-O-Benzoyl-(+)-cyanidan-3-ol vom F=132—135°C.

Beispiel 10

Analog zu Beispiel 9 jedoch ausgehend von 115,8 g 5,7,3′,4′-tetra-O-(4-Fluor-benzoyl)-(+)-cyanidan-3-ol erhält man das 3-O-(4-Fluor-benzoyl)-(+)-cyanidan-3-ol vom F=131—133°C.

Beispiel 11

Wie in Beispiel 9 stellt man das Palladiumschwarz ausgehend von 40 g Palladiumchlorid suspendiert in 4,5 Liter Methanol her, ersetzt das Methanol durch Aethylacetat und hydriert 130 g 5,7,3′,4′-tetra-O-benzyl-3-O-(3,4-dibenzoyloxy-benzoyl)-(+)-cyanidan-3-ol, gelöst in 5,5 1 Aethylacetat. Man

entfernt die organischen Lösungsmittel durch Azeotropie mit Wasser, löst den Rückstand in 500 ml Wasser und lyophilisiert bis zu Gewichtskonstanz. Man erhält so 3-O-protocatechyl-(+)-cyanidan-3-ol vom F=166—168°C.

Beispiel 12

Man verfährt wie in Beispiel 2 beschrieben, und erhält aus 24,5 g 5,7,3',4'-O tetra-Benzyl-3-O-acetylsalicylyl-(+)-cyanidan-3-ol das 3-O-Acetylsalicylyl-(+)-cyanidan-3-ol vom F=118—119°C.

Beispiel 13

Analog Beispiel 1 stellt man Palladiumschwarz aus 24 g Palladiumchlorid in 3,6 1 Methanol her und hydriert 64 g 5,7,3'-4'-O-tetra-Benzyl-3-(2-carboxy-benzoyl)-(+)-cyanidan-3-ol in 3.5 1 Aethylacetat. Nach dem Abfiltrieren des Katalysators und dem Eindampfen der organischen Lösungsmittel nimmt man den Rückstand in 2 Liter Aethanol auf, engt die Lösung auf ca. 100 ml ein, wiederholt dies zwei Mal und trocknet dann den Rückstand bei 40°C und im Hochvakuum bis zu Gewichtskonstanz. Man erhält so das 3-O-(2-Carboxy-benzoyl)-(+)-cyanidan-3-ol vom F=139—142°C.

Beispiel 14

Man stellt wie in Beispiel 1 beschrieben Palladiumschwarz her, tauscht dann das Methanol durch Aethylacetat aus und hydriert 30,8 g 5,7,3',4'-tetra-O-Benzyl-3-(N-phenylcarbamoyl)-(+)-cyanidan-3-ol in 1600 ml Aethylacetat. Nach dem Abfiltrieren des Katalysators, dampft man auf ein Volumen von ungefähr 50 ml ein, gibt 250 ml Wasser zu und stellt mit einer wässrigen Natriumhydroxyd-Lösung (1n) ein pH von 6 ein. Man dampft im Vakuum bei 40°C ein, dann fügt man 500 ml Wasser zu und dampft erneut ein. Man wiederholt dies 3 Mal, löst den Rückstand in 1 Liter Aethanol und dampft erneut ein. Man wiederholt dies, nimmt dann in einer Mischung von 300 ml Aethanol und 300 ml Wasser auf, dampft ein und nimmt endlich in 500 ml Wasser auf. Man dampft auf ein Volumen von ungefähr 100 ml ein, filtriert den entstandenen Niederschlag ab und trocknet im Vakuum zu Gewichtskonstanz. Man erhält so das 3-O-(N-phenylcarbamoyl)-(+)-cyanidan-4-ol vom F=189—200°C.

Beispiel 15

Man verfährt wie in Beispiel 2 beschrieben, jedoch ausgehend von 18,2 g 5,7,3',4'-tetra-O-Benzyl-3-O-methansulfonyl-(+)-cyanidan-3-ol, das man in 600 ml Aethylacetat hydriert. Man destilliert das Lösungsmittel bei Raumtemperatur ab und trocknet den Rückstand bei Raumtemperatur im Hochvakuum. Der Rückstand wird in 500 ml Aethanol bei Raumtemperatur aufgenommen, dann engt man im Hochvakuum bei 0°C auf ungefähr 50 ml ein, wiederholt dies zweimal, dann trocknet man bei 35°C während 15 Minuten im Vakuum und führt das Produkt zu Gewichtskonstanz im Hochvakuum bei Raumtemperatur. Man erhält so das 3-O-Methansulfonyl-(+)-cyanidan-3-ol. Diese Substanz zersetzt sich langsam bereits bei Raumtemperatur; sie kann im Kühlschrank bei —30°C mehrere Tage aufbewahrt werden.

Beispiel 16

Wie im Beispiel 7 beschrieben, hydriert man in einem 3 Liter-Kolben eine Lösung von 40 g 5,7,3',4'-tetra-O-Benzyl-3-O-(4-methyl-benzolsulfonyl)-(+)-cyanidan-3-ol in 1,8 1 Aethylacetat mit 20 g Palladiumkohle (10%). Die Hydrierung dauert 2 1/2 Stunden. Man filtriert vom Palladium und der Kohle ab, dampft das Filtrat im Vakuum bei Raumtemperatur zur Trockne, fügt 1,7 1 Aethanol zu, und destilliert bei 0°C im Hochvakuum. Wenn das Volumen ungefähr 100 ml erreicht hat, fügt man 800 ml Aethanol zu und destilliert weiter bei 0°C. Der Rückstand wird während 15 Minuten im Hochvakuum auf 35°C erwärmt, und bei dieser Temperatur behalten bis zu Gewichtskonstanz. Man erhält so das 3-O-(4-Methyl-benzolsulfonyl)-(+)-cyanidan-3-ol. Um eine Zersetzung zu verhindern wird es im Kühlschrank bei —30°C aufbewahrt.

Beispiel 17

In einem 10 Liter Kolben hydriert man eine Suspension von 29 g Palladiumchlorid in 2,9 Liter Methanol. Man dekantiert das Methanol, wäscht das gebildete Paladiumschwarz zuerst drei Mal mit 300 ml Methanol, dann drei Mal mit 300 ml Aethylacetat. Man gibt dann eine Lösung von 54,5 g 5,7,3',4'-tetra-O-Benzyl-3-O-(2-cyano-äthyl)-(+)-cyanidan-3-ol in 2,9 1 Aethylacetat zu und hydriert. Nach dem Abfiltrieren des Katalysators dampft man das Lösungsmittel unter vermindertem Druck ab, löst den Rückstand in 1,5 1 Wasser und dampft am Rotavopor bei 40°C unter Vakuum auf ein Volumen von ungefähr 500 ml ein. Dies wird zweimal wiederholt, dann lyophilisiert man bis zu Gewichtskonstanz. Man erhält so das 3-O-(2-cyan-äthyl)-(+)-cyanidan-3-ol. Ausbeute 25 g (gleich 94% der Theorie).

Beispiel 18

Man gibt in einen 10 Liter Kolben 56 g 5,7,3',4'-tetra-O-Benzyl-3-(2-cyano-äthyl)-(+)-cyanidan-3-ol, 4 Liter abs. Aethanol und 100 ml Chloroform. Unter Ausschluss von Feuchtigkeit erwärmt man auf 60°C bis zur vollständigen Lösung, dann lässt man auf Raumtemperatur abkühlen und füllt den Kolben mit Argon. Unter Argon gibt man 25 g Palladiumkohle (10%) zu und hydriert. Es werden ungefähr 12

Liter Wasserstoff verbraucht. Man ersetzt dann den Wasserstoff durch Stickstoff, filtriert den Katalysator ab, und dampft den Rückstand unter mittlerem Vakuum ein. Der Rückstand wird mit 1 Liter Aethylacetat verarbeitet und die erhaltene Suspension während 30 Minuten bei 40°C gerührt. Man dampft das Lösungsmittel bis auf ein Volumen von ungefähr 200 ml ein, kühlt den Rückstand auf ungefähr 0°C, filtriert und wäscht den Rückstand mit Aethylacetat. Die Behandlung mit Aethylacetat wird zweimal wiederholt, dann wäscht man den Rückstand zweimal mit Aethylacetat und zweimal mit Methylenchlorid und trocknet unter Hochvakuum bei Raumtemperatur während 24 Stunden. Das Produkt wird in 2 Liter Wasser aufgenommen, dann unter vermindertem Druck auf ein Volumen von ungefähr 200 ml eingeengt. Man gibt 1,8 Liter Wasser zu und engt wiederum auf ein Volumen von ungefähr 200 ml ein. Diese Lösung wird dann bis zu Gewichtskonstanz lyophilisiert. Man erhält so das Chlorhydrat des 3-O-(3-aminopropyl)-(+)-cyanidan-3-ols. F=195°C (unter Zersetzung).

Beispiel 19

In einem 500 ml Kolben, der mit einem aufsteigenden Kühler, welcher ein Chlorkalziumrohr trägt, einem Einfüllstutzen mit Druckausgleich, einer Kanüle zur Einfügen von Stickstoff, einem Thermometer und einem Rührer ausgerüstet ist, gibt man 5,0 g Natriumhydrid (ca. 55%) in einer Oel-suspension. Man lässt während 5 Minuten einen starken Stickstoffstrom durchfliessen, reduziert dann den Stickstoffstrom so, dass man tropfenweise 60 ml wasserfreies Tetrahydrofuran zutropfen kann. Die Mischung wird mittels einem äusseren Oelbad auf 40°C erwärmt, man fügt dann tropfenweise und unter Rühren 7,5 ml (17 g) frisch destilliertes Methyljodid zu. Man fügt dann im Laufe einer Stunde tropfenweise eine Lösung von 52,0 g 5,7,3',4'-tetra-O-Benzyl-(+)-cyanidan-3-ol in 170 ml Tetrahydrofuran zu. Unter Rühren lässt man während 1 1/2 Stunden reagieren, wobei die Temperatur bei 40°C gehalten wird. Man kühlt auf Raumtemperatur ab, gibt 200 ml Wasser zu und rührt während 15 Minuten. Man filtriert und gibt das Filtrat in einen Dekantierkolben der 200 ml Toluol und 200 ml Wasser enthält. Man trennt die organische Phasen und wäscht mit 50 ml Wasser. Die wässrigen Phasen werden 2 Mal mit 400 ml Methylenchlorid extrahiert, die vereinigten organsichen Phasen über Magnesiumsulfat getrocknet und unter mittlerem Vakuum bei 40°C eingedampft. Der Rückstand wird während der Nacht bei 15 mm Hg und 45°C getrocknet. Dieser Rückstand wird bei 100°C in 510 ml Methoxyäthanol aufgenommen, heiss filtriert, und langsam unter Rühren abgekühlt. Nach 8 Stunden, wird er gebildete Niederschlag abfiltriert, mit 30 ml kaltem Methoxyäthanol, das vorher bei 50°C im Vakuum während einer Nacht getrocknet wurde, gewaschen. Man erhält so das 5,7,3',4'-tetra-O-Benzyl-3-O-methyl-(+)-cyanidan-3-ol vom F=124—125°C.

Beispiel 20

In einen 6 Liter Kolben, der mit einem mechanischen Rührer und einem aufsteigenden Kühler ausgerüstet ist, gibt man eine wässrige Lösung von 1,5 Liter 50%iger Natronlauge, eine Lösung von 52 g von 5,7,3',4'-tetra-O-Benzyl-(+)-cyanidan-3-ol in 1,5 Liter Chlorbutyl und 6,8 g Tetrabutylammoniumhydrogensulfat. Man wärmt auf 50°C und hält bei dieser Temperatur während 2 h 45 unter starkem Rühren. Nach dem Abkühlen auf Raumtemperatur trennt man die beiden Phasen, wäscht die organische Phase dreimal mit 300 ml Wasser und trocknet über Magnesiumsulfat. Man filtriert und destilliert das Chlorbutyl ab. Man kristallisiert den Rückstand zweimal in 3,2 Liter abs. Aethanol. Nach dem Trocknen erhält man das 5,7,3'-4'-O-tetra-Benzyl-3-O-butyl-(+)-cyanidan-3-ol vom F=55—56°C.

Beispiel 21

In einem 500 ml Kolben, ausgerüstet mit einem Druckeinlassstutzen, einem Kühler, der ein Kalziumchloridrohr trägt, einem Stickstoffeinlassrohr und einem magnetischen Rührer, gibt man unter Stickstoff und Rühren 52 g 5,7,3',4'-tetra-O-Benzyl-(+)-cyanidan-3-ol in 360 ml wasserfreiem Pyridin, und fügt 17 g Butyrylchlorid zu. Man lässt die Mischung bei Raumtemperatur unter Rühren während 5 1/2 Stunden reagieren, dann gibt man 500 ml Wasser enthaltend Eisstücke zu. Man rührt die Mischung während einer Stunde und trennt die ölige Schicht ab. Diese wird zurest mit 500 ml einer wässrigen Lösung von Natriumhydrogenkarbonat (1n), dann zwei Mal mit 500 ml Wasser gerührt. Das feste Produkt, das sich bildet, wird mit Wasser gewaschen und im Vakuum getrocknet. Man kristallisiert den Rückstand in einer Mischung von 3 Liter Aethanol und 400 ml Aceton. Man erhält so das 5,7,3',4'-tetra-O-Benzyl-3-O-butyryl-(+)-cyanidan-3-ol, das nach dem Trocknen bei 92—93°C schmilzt.

Beispiel 22

Analog Beispiel 21, jedoch ausgehend von 21.5 g 3,3-dimethylbutanoyl-chlorid erhält man das 5,7,3',4'-tetra-O-benzyl-3-O-(3,3-dimethyl-butanoyl)-(+)-cyanidan-3-ol vom F=58°C. Die Reaktionsdauer ist eine Stunde.

Beispiel 23

In einen 1 Liter Kolben mit einem Kühler, welcher ein Chlorkalziumrohr trägt, gibt man 52 g 5,7,3',4'-tetra-O-Benzyl-(+)-cyanidan-3-ol, 400 mg Triäthylamin, 80 ml Pyridin und 16 g Succinanhydrid. Die Mischung wird am Rückflusskühler während einer Stunde erwärmt, dann entfernt man im

Vakuum bei 40°C die Lösungsmittel, wäscht den Rückstand zweimal mit 300 ml Wasser, das mit Salzsäure auf ein pH 3 gebracht wurde, dann zweimal mit destilliertem Wasser. Man kristallisiert zweimal in 3 Liter Aethanol. Man erhält so das 5,7,3',4'-tetra-O-Benzyl-3-O-(3-carboxy-propionyl)-(+)-cyanidan-3-ol, das bei 95—96°C schmilzt.

Beispiel 24

Analog Beispiel 21, jedoch in einem 1 Liter Kolben und ausgehend von 104 g 5,7,3',4'-tetra-O-Benzyl-(+)-cyanidan-3-ol in 720 ml Pyridin und in Gegenwart von 61 g Caproylchlorid, erwärmt man die Mischung auf 50°C unter Rühren während 1 1/2 Stunden, giesst dann auf 3 Liter einer Eis-Wassermischung, rührt 1 Stunde, filtriert den gebildeten Niederschlag ab, wäscht in zuerst zweimal mit 1 Liter (1n) wässrige Natronlauge, dann zweimal mit einem Liter Wasser und trocknet im Vakuum bei Raumtemperatur über Phosphorpentoxyd. Dann kristallisiert man aus 1,4 1 Methoxyäthanol und erhält so das 5,7,3',4'-tetra-O-benzyl-3-O-decanoyl-(+)-cyanidan-3-ol vom F=88—89°C.

Beispiel 25

Man arbeitet wie im Beispiel 21 unter Verwendung von 44 g Palmitoylchlorid und unter Erhitzen der Reaktionsmischung auf 90°C während zwei Stunden. Man reinigt das erhaltene 5,7,3',4'-tetra-O-Benzyl-3-O-palmitoyl-(+)-cyanidan-3-ol durch Lösen in der Wärme in 80 ml Aceton und tropfenweisem Zufügen zu dieser Lösung von 3 Liter Aethanol. F=57—59°C.

Beispiel 26

Man erarbeitet wie in Beispiel 23, benützt jedoch 24.5 g Tetrahydrophthalsäureanhydrid. Die Reaktionsmischung wird 45 Minuten am Rückflusskühler gekocht, das Lösungsmittel unter mittlerem Vakuum bei 40°C abdestilliert. Der Rückstand wird in 600 ml Methylenchlorid aufgenommen und zweimal mit 500 ml 2n-Salzsäure und zweimal mit 500 ml dest. Wasser extrahiert. Man trocknet die Methylenchlorid-Lösung über Magnesiumsulfat und dampft das Lösungsmittel ab. Der Rückstand wird aus einer Mischung von Chlorkohlenstoff und Petroläther umkristallisiert. Man erhält so das 5,7,3',4'-tetra-O-Benzyl-3-O-tetrahydrophthalyl-(+)-cyanidan-3-ol vom F=67—68°C.

Beispiel 27

Man verfährt wie in Beispiel 21, jedoch unter Verwendung von 22,5 g Benzoylchlorid und erwärmt die Reaktionsmischung 6 Stunden auf 80°C. Man wäscht mit Natriumhydrogencarbonat wobei sich ein festes Produkt ausscheidet und kristallisiert dieses aus einer Mischung von 7 Liter Aethanol und 1,25 Liter Aceton. Man erhält so das 5,7,3',4'-tetra-O-Benzyl-3-O-benzoyl-(+)-cyanidan-3-ol vom Schmelzpunkt F=115—116°C.

Beispiel 28

Man verfährt wie in Beispiel 21, jedoch unter Verwendung von 25,4 g 4-Fluor-benzoylchlorid. Man hält die Reaktionsmischung 7 Stunden bei Raumtemperatur. Das feste Produkt wird in 3,5 Liter Aceton warm gelöst und durch Zufügen von 800 ml Wasser ausgefällt. Man erhält so das 5,7,3',4'-tetra-O-Benzyl-3-O-(4-fluor-benzoyl)-(+)-cyanidan-3-ol vom Schmelzpunkt 154°C.

Beispiel 29

Man verfährt wie in Beispiel 21, jedoch unter Verwendung von 56,5 g 3,4-Dibenzyloxy-benzoylchlorid in 130 ml Pyridin. Das Reaktionsgemisch wird bei Raumptemperatur während 2 h 45 gerührt. Das erhaltene feste Produkt wird zweimal aus Aceton umkristallisiert. Man erhält so das 5,7,3',4'-tetra-O-Benzyl-3-O-(3,4-dibenzyloxy-benzoyl)-(+)-cyanidan-3-ol vom Schmelzpunkt 162—163°C.

Beispiel 30

Man verfährt wie in Beispiel 21, jedoch unter Verwendung einer Lösung von 32 g Acetylsalicylylchlorid in 180 ml Pyridin. Das Reaktionsgemisch wird 2 Stunden bei 65°C gerührt. Nach dem Waschen mit Natriumhydrogencarbonat und mit Wasser kristallisiert man das feste rohe Produkt einmal mit 8 Liter Aethanol, dann mit einer Mischung von 5 Liter Aethanol und 1,2 Liter Aceton. Man erhält so das 5,7,3',4'-tetra-O-Benzyl-3-O-(acetylsalicylyl)-(+)-cyanidan-3-ol vom Schmelzpunkt 136—137°C.

Beispiel 31

In einem 6 Liter Kolben, ausgerüstet mit einem mechanischen Rührer, einer Rückflusschlange mit einem Calziumchloridrohr, einem Stickstoffeinlassstutzen und einem Thermometer, gibt man 260 g 5,7,3',4'-tetra-O-Benzyl-(+)-cyanidan-3-ol, 89 g Phthalsäureanhydrid, 3 Liter Triäthylamin und 1 Liter Pyridin. Unter Stickstoff wird diese Mischung am Rückflusskühler gekocht (92°C) während 9 Stunden, dann lässt man auf Raumtemperatur abkühlen und filtriert. Man dampft die Mischung im Vakuum bei 40°C am Rotavapor ein, nimmt den öligen Rückstand in 500 ml Aethylenchlorid auf und extrahiert zweimal mit 500 ml 2n wässriger Salzsäure und zweimal mit 500 ml Wasser. Die organische Lösung wird über Magnesiumsulfat getrocknet, filtriert und zur Trockne eingedampft. Das rohe Produkt wird

aus 2,5 Liter Toluol und 1,6 Liter Ligroin umkristallisiert. Man erhält so das 5,7,3',4'-tetra-O-Benzyl-3-O-(2-carboxy-benzoyl)-(+)-cyanidan-3-ol vom F=127—129°C.

Beipsiel 32

Man verfährt wie in Beispiel 21, jedoch in einem 1 Liter Kolben und unter Verwendung von 65g 5,7,3',4'-tetra-O-Benzyl-(+)-cyanidan-3-ol in 800 ml Pyridin. Man kocht unter Stickstoff bei 100°C und fügt tropfenweise 17,1 ml (18,7 g) M-Phenylisocyanat zu. Man lässt 1 Stunde bei 100°C, kühlt dann auf Raumtemperatur ab, filtriert und fügt 6 Liter Wasser unter starkem Rühren zu, wobei sich ein weisses Pulver bildet. Man filtriert, wäscht den Rückstand mit 6 Liter Wasser und trocknet im Vakuum bei 60°C während 2 Tagen. Man kristallisiert aus 8 Liter Aceton und erhält das 5,7,3',4'-tetra-O-Benzyl-3-O-(M-Phenyl-carbamoyl)-(+)-cyanidan-3-ol vom F=204°C.

Beispiel 33

In einem 750 ml Kolben der mit einem magnetischen Rührer, einem Calziumchloridrohr, einem Einlassstutzen und einer Stickstoffzuführung ausgerüstet ist, gibt man 65 g 5,7,3',4'-tetra-O-Benzyl-(+)-cyanidan-3-ol gelöst in 500 ml Pyridin und 25 ml Triäthylamin. Man lässt einen Stickstoffstrom durchfliessen und kühlt auf —15°C und gibt innerhalb von 30 Minuten 23 g Methansulfonylchlorid zu. Man rührt das Reaktionsgemisch 30 Minuten bei —15°C, dann giesst man auf 2,5 Liter eines Eis-Wassergemisches, filtriert den Niederschlag ab, rührt mit einem Liter Wasser und filtriert. Das erhaltene Produkt wird nach dem Trocknen über Phosphorpentoxyd aus 2 Liter n-Butanol umkristallisiert, filtriert, mit 250 ml Methanol, dann mit 500 ml Methanol gewaschen. Die Substanz wird bei Raumtemperatur im Vakuum über Phosphorpentoxyd getrocknet. Man erhält so das 5,7,3',4'-tetra-O-Benzyl-3-O-methansulfonyl-(+)-cyanidan-3-ol vom F=138—139°C.

Beispiel 34

In einen 500 ml Kolben, ausgerüstet mit einem Rückflusskühler, der ein Calziumchloridrohr trägt, einer Stickstoffzuführung, einem Thermometer und einem mechanischen Rührer gibt man 158 g 5,7,3',4'-tetra-O-Benzyl-(+)-cyanidan-3-ol und 250 ml Pyridin. Man erwärmt die Mischung auf 90°C, wobei sich die Mischung löst und fügt dann heiss 67 g 4-Methyl-benzol-sulfonylchlorid zu und hält die Lösung unter Stickstoff und Rühren bei 90°C während 4 Stunden. Man kühlt dann auf —30°C ab, lässt 2 1/2 Stunden stehen wobei sich ein Niederschlag bildet. Man fügt 1,5 Liter Wasser zu, rührt die Mischung, filtriert den Niederschlag ab, wäscht ihn mit 1n Lösung von wässrigem Natriumhydrogencarbonat, dann mit Wasser bis das Filtrat neutral ist und kristallisiert aus 5 Liter Chloroform und 20 Liter Isopropanol um. Man erhält so das 5,7,3',4'-tetra-O-Benzyl-3-O-(4-Methylbenzolsulfonyl)-(+)-cyanidan-3-ol vom F=173—174°C.

Beispiel 35

In einen 20 Liter Kolben mit einer Dekantierampulle und einem mechanischen Rührer gibt man langsam und unter Rühren: zuerst eine Lösung von 300 g 5,7,3',4'-tetra-O-Benzyl-(+)-cyanidan-3-ol in 3 Liter Toluol, dann 4,5 Liter einer wässrigen Lösung von Natriumhydroxid (50%ig) und dann 16 g Triton B. Man rührt weiter und gibt dann tropfenweise in 45 Minuten 82 g Acrylnitril zu. Man rührt stark während 2 Stunden bei Raumtemperatur, gibt dann 30 g Acrylnitril zu und rührt weiter während einer Nacht. Eine 3. Portion von 100 g Acrylnitril wird dann zugegeben. Man rührt während 2 weiteren Stunden, isoliert die Toluolphase, wäscht 3 Mal mit 1 Liter 0,1n Salzsäure und dann mit 2 Liter Wasser. Die Toluolphase wird über Magnesiumsulfat getrocknet und das Toluol abgedampft. Den Rückstand nimmt man in 1,8 Liter Aceton auf und gibt diese Lösung unter starkem Rühren zu 5,4 Liter kochendem Aethanol. Das 5,7,3',4',-tetra-O-Benzyl-3-O-(2-cyano-äthyl)-(+)-cyanidan-3-ol fällt beim Erkalten aus, es wird filtriert und getrocknet. F=100—101°C.

Beispiel 36

In einen 500 ml Kolben, der eine Stickstoffeinführung, einen Zuführstutzen der druckkompensiert ist, einem Calziumchloridrohr, und einem magnetischen Rührer ausgerüstet ist gibt man 52 g 5,7,3',4'-tetra-O-benzyl-(+)-cyanidan-3-ol, 260 ml wasserfreies Dioxan und 130 mg Paratoluolsulfonsäure. Zu dieser Mischung gibt man unter Rühren langsam 26 ml (24 g) Dihydropyran. Man rührt bei Raumtemperatur während 2 1/2 Stunden, filtriert und dampft das Filtrat im Vakuum ein. Der Rückstand wird in 500 ml Chloroform aufgenommen, die erhaltene Lösung erst zweimal 300 ml einer 1-n Lösung vom wässrigen Hydrogencarbonat, dann mit 300 ml destilliertem Wasser gewaschen. Man trocknet die Chloroformlösung über Magnesiumsulfat, filtriert und dampft ein. Der Rückstand wird im Hochvakuum bei Raumtemperatur getrocknet. Man erhält so das 5,7,3',4'-tetra-O-Benzyl-3-O-tetrahydrapyranyl-(+)-cyanidan-3-ol, als Mischung von 2 Diastereoisomeren. Das Produkt ist eine klebrige Masse deren Schmelzpunkt nicht bestimmt werden kann.

Beispiel 37

Unter Stickstoff und gutem Rühren stellt man eine Suspension von 92,8 g einer 55%igen Dispersion von Natriumhydrid in Oel (51 g NaH) in einem Liter frisch destilliertem Dimethylformamyd her.

Man kühlt sie auf eine Temperatur zwischen —5 und 0°C ab und gibt dazu eine Lösung von 145 g (+)-Cyanidan-3-ol in 2 Liter frisch destilliertem Dimethylformamyd zu, so dass die Dispersionstemperatur konstant bleibt. Entstehender Wasserstoff wird durch zirkulierenden Stickstoff entfernt.

Nach der Einführung vom (+)-Cyanidan-3-ol lässt man bei tiefer Temperatur während 15 Minuten stehen, gibt dann im Laufe von 1 1/2 Stunden 276,5 ml Benzylbromid zu, so dass die Temperatur nicht über 0°C steigt. Man lässt bei dieser Temperatur nochmals eine halbe Stunde stehen und dann auf Raumtemperatur erwärmt.

Man destilliert im Vakuum bei 60°C das Dimethylformamid ab, nimmt den öligen Rückstand in 3 Liter Trichloräthylen auf, wäscht die organische Lösung mit 3 Liter destilliertem Wasser und filtriert. Man engt ein und kühlt ab und erhält so das 5,7,3',4'-tetra-O-Benzyl-(+)-cyanidan-3-ol das in weissen Nadeln kristallisiert. F=144—145°C.

Beispiel 38

Unter den gleichen Bedingungen wie in Beispiel 37, jedoch unter Verwendung von Dimethylformamid das sorgfältig getrocknet worden ist, erhält man das 5,7,3',4'-tetra-O-Benzyl-(+)-cyanidan-3-ol.

Beispiel 39

Analog Beispiel 37, jedoch unter Verwendung von 2,25 Mol Benzylchlorid, erhält man das Tetra-O-benzyl-5,7,3',4'-(+)-cyanidan-3-ol.

Beispiel 40

Zu einer Lösung von 0,93 g Natriumhydriddispersion in Oel (0,51 g NaH) in 10 ml Dimethylsulfoxyd das frisch destilliert wurde, gibt man langsam unter Rühren und bei Raumtemperatur gibt man eine Lösung von 1,45 g (+)-Cyanidan-3-ol in 20 ml frisch destilliertem Dimethylsulfoxyd. Der entstehende Wasserstoff wird durch zirkulierenden Stickstoff entfernt. Nach einer Stunde Rühren bei Raumtemperatur hört die Stickstoffentwicklung praktisch auf und das Natriumsalze des (+)-Cyanidan-3-ols ist zum Teil an der Kolbenwand abgeschieden. Man gibt dann tropfenweise 2,68 ml Benzylbromid zu. Man lässt 1 1/2 Stunden unter Rühren bei Raumtemperatur stehen, gibt die Reaktionslösung langsam und unter Rühren in 300 ml einer kalten wässrigen, 10%igen Lösung von Kochsalz. Der Niederschlag wird abfiltriert, und im Vakuum bei 80°C getrocknet, dann aus 40 ml Tetrachlorkohlstoff umstrisallisiert. Man erhält 1,96 g 5,7,3',4'-tetra-O-Benzyl-(+)-cyanidan-3-ol.

Beispiel 41

Man verfährt wie in Beispiel 37, verwendet jedoch 416 g $\alpha$-Brom-o-xylen anstelle von Benzylbromid. Der Rückstand der nach dem Abdampfen des Dimethylformamid erhalten wird, wird in Chloroform aufgenommen, die Lösung mit destilliertem Wasser gewaschen, filtriert und eingedampft. Man kristallisiert den Rückstand aus einer Mischung von Benzol und Petroläther (6:3 v/v). Man erhält so das 5,7,3',4'-tetra-O-(2-methyl-benzyl)-(+)-cyanidan-3-ol vom F 88—90°C.

Beispiel 42

Analog Beispiel 37, jedoch unter Verwendung von 416 g $\alpha$-Brom-p-xylol anstelle von Benzylbromid erhält man das 5,7,3',4'-tetra-O-(4-methyl-benzyl)-(+)-cyanidan-3-ol das nach Umkristallisieren aus Tetrachlorkohlenstoff bei 66—70°C schmilzt.

Beispiel 43

Analog Beispiel 37, aber unter Verwendung von 562 g 2-Brombenzylbromid anstelle von Benzylbromid erhält man das 5,7,3',4'-tetra-O-(2-Brom-benzyl)-(+)-cyanidan-3-ol, das aus einer Mischung von Chloroform und Tetrachlorkohlenstoff (40:55 v/v) umkristallisiert wird. F=155—157°C.

Beispiel 44

Analog Beispiel 37, jedoch unter Verwendung von 181 g Chlordimethyläther anstelle von Benzylbromid erhält man das 5,7,3',4'-tetra-O-Methoxymethyl-(+)-cyanidan-3-ol, das nach Umkristallisieren aus einer Mischung von Wasser mit Methanol (1:1 v/v) bei 92—93°C schmilzt.

Beispiel 45

Analog Beispiel 37, jedoch unter Verwendung von 272 g Allylbromid anstelle Benzylbromid erhält man das 5,7,3',4'-tetra-O-Allyl-(+)-cyanidan-3-ol das nach dem Umkristallisieren aus n-Hexan bei 78,5—80°C schmilzt.

Beispiel 46

Analog Beispiel 37, jedoch unter Verwendung von 244 g Aethylchloroformiate anstelle von Benzylbromid erhält man das 5,7,3',4'-tetra-O-Aethoxycarbonyl-(+)-cyanidan-3-ol dessen Schmelzpunkt ungefähr bei 65°C liegt.

Beispiel 47

Man löst 1,45 g (+)-cyanidan-3-ol in 50 ml wasserfreiem Aceton, gibt 6,91 g wasserfreies Kaliumcarbonat zu und erhitzt ihn am Rückflüss im Stickstoff. Zu dieser lösung gibt man eine Lösung von 6,26 g p-Brom-phenacyl-bromid in 30 ml wasserfreiem Aceton und lässt weiter kochen unter mechanischem Rühren während 3 Stunden. Nach dem Abkühlen der Reaktionsmischung filtriert man die Salze ab und dampft die Lösung zur Trockne ein. Den Rückstand nimmt man in 50 ml Chloroform auf, wäscht die Chloroformlösung 3 Mal mit 50 ml Wasser, dampft das Chloroform ab, löst den Rückstand in ungefähr 1000 ml Aether, filtriert und engt die Aetherlösung auf 150 ml ein. Man erhält so das 5,7,3',4'-tetra-O-(4-Brom-phenacyl)-(+)-cyanidan-3-ol, das nach dem Umkristallisieren auf Aethanol bei 127—129°C schmilzt.

Beispiel 48

In einem 500 ml Kolben der mit einem Rückflusskühler, welcher eine Calziumchloridrohr trägt, einer Stickstoffeinführung, und einem Zuflussstutzen und einem magnetischen Rüher ausgerüstet ist, gibt man unter Stickstoff 5,4 g Natriumhydrid in Oelsuspension (55%), wäscht 2 Mal mit 50 ml wasserfreiem Hexan zum Entfernen des Oels, trocknet das Natriumhydrid im Vakuum und gibt eine Lösung von 32,5 g 5,7,3',4'-tetra-O-Benzyl-(+)-cyanidan-3-ol in 200 ml Tetrahydrofuran (wasserfrei) zu. Man erwärmt 2 Stunden am Rückfluss, kühlt die Mischung in einem Eisbad ab und fügt tropfenweise in 45 Minuten eine Lösung von 9 g Methoxymethylchlorid in 40 ml wasserfreiem Tetrahydrofuran zu. Man rührt im Eisbad während 2,5 Stunden, filtriert die Lösung über Cellit und dampft bei Raumtemperatur am Rotavapor zum Trocknen. Man kristallisiert den Rückstand 2 Mal aus einer Mischung von 200 ml Aceton und 500 ml 2-Propanol. Das erhaltene 5,7,3',4'-tetra-O-Benzyl-3-O-methoxymethyl-(+)-cyanidan-3-ol schmilzt bei 108—110°C.

Beispiel 49

In einem 5 Liter Kolben hydriert man 7,15 g Palladiumchlorid suspendiert in 2,4 Liter Methanol. Der grosse Teil des Methanols wird abdekantiert, das Palladium 4 Mal mit 4 Liter Methanol, und 4 Mal mit 200 ml Aethylacetat gewaschen. Beim letzten Dekantieren lässt man das Palladium unter einer dünnen Schicht Aethylacetat. Man fügt eine Lösung von 20 g 5,7,3',4', tetra-O-Benzyl-3-O-methoxymethyl-(+)-cyanidan-3-ol in 2,4 Liter Aethylacetat. Die Hydrierung verbraucht 3,6 Liter Wasserstoff und dauert 5 Stunden. Man filtriert dann das Palladium ab, wäscht 2 Mal mit 200 ml Aethylacetat, dampft die vereinigten Aethylacetatlösungen ein, gibt 700 ml Aethanol zum Rückstand, dampft auf ungefähr 100 ml ein und wiederholt ein zweites Mal und dann 2 Mal mit 700 ml Wasser. Der Rückstand wird im Hochvakuum bei Raumtemperatur zur Gewichtskonstanz getrocknet. Man erhält das 3-O-Methoxymethyl-(+)-cyanidan-3-ol vom F=106—109°C.

Beispiel 50

In einem Kolben A, der mit einer Stickstoffeinführung, einem Einlassstutzen und einem Vakuumanschluss ausgerüstet ist, gibt man 42,5 g Trifluorborätherat in 50 ml frisch über Natrium destilliertes Diglym. In den Einfüllstutzen gibt man ein 7,65 g Natriumborhydrid in 250 ml Diglym.

In einem zweiten Kolben B, der mit einem Einfüll-stutzen, einer Kanüle die mit der Vakuumkanüle des Kolben A verbunden ist, einer unabhängigen Stickstoffzuführung und einem aufsteigenden Kühler dessen obere Oeffnung mit einer Waschflasche enthaltend Aceton, ausgerüstet ist, gibt man eine Lösung von 28 g 5,7,3',4'-tetra-O-Benzyl-3-O-(2-cyan-äthyl)-(+)-cyanidan-3-ol in 500 ml Tetrahydrofuran, das frisch über Natrium destilliert wurde. Die beiden Kolben A und B sind mit magnetischem Rührer ausgerüstet, der Kolbern A wird stark, während der Kolben B schwach gerührt wird. Man lässt ein Stickstoffstrom durch den Kolben A, den Kolben B, den Kühler des Kolben B und der Waschflasche spülen. In den Kolben A gibt man tropfenweise im Laufe 1 1/2 Stunden eine Lösung von Natriumborhydrid, worauf Borhydrid entsteht, das mit dem Stickstoff in den Kolben B transportiert wird. Dort reduziert er die 2-cyan-äthyl Gruppierung zur 3-Amino-propyl Gruppierung. Man lässt nach Beendigung der Natriumborhydrid-Zuleitung während 5 1/2 Stunden reagieren. Durch den Einfüllstutzen des Kolben B gibt man dann 100 ml abs. Aethanols zu, wobei sich eine Gasentwicklung zeigt.

Der Inhalt des Kolbens B wird dann am Rotavapor im Vakuum bei einer Temperatur die kleiner ist als 30°C destilliert. Der Rückstand wird in 200 ml Butanol kochend aufgenommen und dann über Nacht bei 4°C stehen gelassen, wobei sich ein Oel bildet. Man dekantiert das Butanol und wiederholt diese Behandlung 4 Mal.

Die vereinigten Butanol-Extrakte werden am Rotavapor und der Rückstand am Hochvakuum über Phosphorpentoxyd während 24 Stunden, bis zur Gewichtskonstanz getrocknet. Das erhaltene 5,7,3',4'-tetra-O-Benzyl-3-O-(3-amino-propyl)-(+)-cyanidan-3-ol schmilzt bei 93—95°C.

Beispiel 51

Unter Stickstoff und starkem Rühren stellt man eine Suspension von 65,5 g einer Natriumhydriddispersion in Oel (55%) in 700 ml frisch destilliertem Formamid her. Man kühlt diese Suspension auf —5 bis 0°C, fügt langsam eine Lösung von 650 g 5,7,3',4'-tetra-O-Benzyl-(+)-cyanidan-3-ol in 3800 ml

frisch destilliertem Dimethylformamid so zu, dass die Temperatur zwischen –5 und 0°C bleibt. 15 Minuten später gibt man in ungefähr 45 Minuten 178,5 ml Benzylbromid so zu, dass die Temperatur immer unter 0°C bleibt. Man hält weitere 30 Minuten diese Temperatur, lässt unter Rühren auf Raumtemperatur erwärmen, destilliert das Dimethylformamid im Vakuum bei 60°C am Rotavapor ab, nimmt den Rückstand bei Raumtemperatur bei 3 Liter Chloroform auf, wäscht dann 3 Mal mit 2 Liter Wasser, filtriert die Chloroformlösung und dampft sie ein. Der Rückstand wird in 1750 ml heissem Benzol aufgenommen und man fügt langsam in der Wärme und unter Rühren Petroläther bis zu beginnender Trübung (es ist ca. 1 Liter Petroläther notwendig). Nach dem Abkühlen erhält man das 3,5,7,3',4'-penta-O-Benzyl-(+)-cyanidan-3-ol vom F=119—121°C.

Beispiel 52

In einem 50 ml Kolben mit einem Zufüllstutzen, einer Stickstoffzuführung und einem Kühler, der ein Calziumchloridrohr trägt, gibt man 0,96 g einer 55%igen Dispersion von Natriumhydrid on Oel und trocknet das Ganze durch Erwärmen im Stickstoffstrom. Nach dem Abkühlen unter einem schwachen Stickstoffstrom gibt man 10 ml frisch destilliertes Dimethylsulfoxyd zu und rührt bei Raumtemperatur. Man fügt tropfenweise eine Lösung von 1,45 g (+)-Cyanidan-3-ol in 15 ml frisch destilliertem Dimethylsulfoxyd zu wobei sich Wasserstoff entwickelt. 30 Minuten später gibt man eine Lösung von 2,74 ml Methoxyäthoxymethylchlorid in 10 ml Dimethylsulfoxyd zu und giesst 30 Minuten später die Reaktionslösung in 150 ml mit Kochsalz gesättigtem Wasser, und stellt pH auf 7. Man erhält ein klebriger braunschwarzer Niederschlag den man 3 Mal mit 100 ml Toluol extrahiert. Die erhaltenen Lösungen werden vereinigt, und 3 Mal mit 50 ml Wasser gewaschen. Man entfernt das Toluol und extrahiert das erhaltene braunrote Oel 10 Mal mit 100 ml n-Hexan am Rückfluss. Die n-Hexan-Lösungen ergeben das 5,7,3',4'-tetra-O-Methoxyäthoxymethyl-(+)-cyanidan-3-ol in Form eines gelbens Oels, das bei 150°C/0,02 mmHg zersetzt wird.

Beispiel 53

In einen 500 ml Kolben, der mit einem Zulaufstutzen, einem Kühler der ein Calziumchloridrohr trägt, einer Stickstoffzuführung und einem magnetischen Rührer ausgerüstet ist, gibt man 52 g 5,7,3',4'-tetra-O-Benzyl-(+)-cyanidan-3-ol in 300 ml wasserfreiem Pyridin. Man rührt unter Stickstoff und fügt tropfenweise 18 g Aethylchloroformiat zu und erhitzt die Mischung 7 Stunden auf 60°C. Man lässt abkühlen, mischt mit 500 ml Eiswasser, filtriert den gebildeten Niederschlag ab, mischt ihn mit 500 ml einer wässrigen 0,5 N-Lösung von Natriumhydrogencarbonat, filtriert den Niederschlag ab, wäscht ihn mit Wasser und trocknet ihn im Vakuum über Nacht. Der Rückstand wird in 550 ml Aceton aufgenommen, dann filtriert, kocht das Filtrat am Rückflusskühler mit 3,3 Liter Aethanol und lässt 2 Tage bei Raumtemperatur stehen. Der Niederschlag wird abfiltriert und aus einer Mischung von 250 ml Aceton und 1,5 Liter Aethanol umkristallisiert. Man erhält so das 5,7,3',4'-tetra-O-Benzyl-3-O-äthoxycarbonyl-(+)-cyanidan-3-ol vom F = 83—84°C.

Beispiel 54

Man stellt wie in Beispiel 1 Palladiumschwarz ausgehend von 4 g Palladiumchlorid her, fügt eine Lösung von 15 g 5,7,3',4'-tetra-O-Benzyl-3-O-äthoxycarbonyl-(+)-cyanidan-3-ol in 600 ml Aethylacetat. Man hydriert während 2 1/2 Stunden wobei 2,33 Liter Wasserstoff verbraucht werden. Man filtriert das Palladium ab, wäscht es 2 Mal mit 200 ml Aethylacetat und dampft die vereinigten Aethylacetatlösungen zur Trockne ein. Zum Rückstand gibt man 500 ml Aethanol, dampft ein auf ein Volumen von ungefähr 50 ml und fügt 200 ml Aethanol und 250 ml Wasser zu, dampft erneut auf ein Volumen von 50 ml, gibt 500 ml Wasser zu und dampft auf 50 ml ein. Man wiederholt diese Operation, filtriert dann den Niederschlag ab, trocknet ihn im Hochvakuum zur Gewichtskonstanz. Man erhält so das 3-O-äthoxycarbonyl-(+)-cyanidan-3-ol vom F = 123—124°C.

Beispiel 55

In einem 750 ml Kolben der mit einem Zulaufstutzen, einer Kühlschlange, die ein Chlorcalziumrohr trägt, einem Stickstoffeinlass und einem magnetischen Rührer ausgerüstet ist gibt man eine Lösung von 65 g 5,7,3',4'-tetra-O-Benzyl-(+)-cyanidan-3-ol in 350 ml wasserfreiem Pyridin. Man erwärmt auf 60°C gibt dann tropfenweise unter Rühren und Stickstoff 31 g Phenylacetylchlorid zu, lässt unter den gleichen Bedingungen 6 Stunden reagieren und giesst die Reaktionsmischung nach dem Abkühlen auf 1 kg gestossenes Eis. Man rührt bis das Eis geschmolzen ist, dekantiert das Wasser, wäscht den Niederschlag 2 Mal mit 1 Liter einer 1N wässrigen Lösung von Natriumhydrogencarbonat, dann 2 Mal mit 1 Liter Wasser. Man trocknet im Vakuum über Nacht und kristallisiert das Produkt in 1,2-Liter Aethoxyäthanol. Das so erhaltene 5,7,3',4'-tetra-O-Benzyl-3-O-phenylacetyl-(+)-cyanidan-3-ol schmilzt bei 83—85°C.

Beispiel 56

In einen 250 ml Kolben, der mit einem Kühler, welcher ein Chlorcalziumrohr trägt, eine Stickstoffeinführung und einem magnetischen Rührer ausgerüstet ist, gibt man 6,5 g 5,7,3',4'-tetra-O-Benzyl-(+)-cyanidan-3-ol, 1,9 g 2,4-Dinitro-fluorbenzol, 3 g Kaliumfluorid, 500 mg 18-Crown-6- und

100 ml wasserfreies Tetrahydrofuran. Man kocht am Rückfluss unter Rühren und Stickstoff während 24 Stunden, lässt abkühlen, fügt 250 ml Toluol zu, wäscht 4 Mal mit 300 ml einer gesättigten wässrigen Lösung von Kochsalz, trocknet die organische Phase über Magnesiumsulfat und dampft die Lösungsmittel unter Vakuum ab. Man wäscht den Rückstand 2 Mal mit 25 ml Petroläther, löst ihn dann in 45 ml Chloroform, gibt langsam 325 ml abs. Aethanol unter Kochen zu. Man lässt erkalten und erhält ein Oel das durch Dekantation abgetrennt wird. Man wäscht mit wenig Aethanol und trocknet im Vakuum während 24 Stunden. Man erhält so das 5,7,3',4'-tetra-O-Benzyl-3-O-(2,4-dinitro-phenyl)-(+)-cyanidan-3-ol vom F = 61—62°C.

Beispiel 57

Man gibt in einen 50 ml Kolben 400 mg Natriumhybrid (55%ige Dispersion in Oel) lässt während 5 Minuten ein starkes Stickstoffstrom einfliessen und fügt 5 ml wasserfreies Tetrahydrofuran, das frisch über Natrium destilliert wurde, zu. Die Mischung wird auf 40°C erwärmt, dann eine Lösung von 0,5 ml Methyljodid in 10 ml wasserfreiem Tetrahydrofuran, dann eine Lösung von 2,3 g 5,7,3',4'-Tetra-O-methoxymethyl-(+)-cyanidan-3-ol in 10 ml wasserfreiem Tetrahydrofuran zu. Die Reaktionsmischung wird bei 40°C während 1 Stunde gerührt, dann filtriert. Zum Filtrat gibt man 20 ml Toluol und 40 ml Wasser, schüttelt und lässt ruhen bevor man die organische Phase abtrennt. Diese wird erneut mit 10 ml Wasser gewaschen. Die Waschwasser werden mit 30 ml Methylenchlorid extrahiert, die organischen vereinigten Extrake über Magnesiumsulfat getrocknet, filtriert, dann am Rotavapor eingedampft. Das zurückbleibende Oel wird in 55 ml Methanol aufgenommen, mit Aktivkohle behandelt, das Methanol verdampft, das zurückbleibende Oel 2 Mal mit 25 ml petroläther in der Wärme extrahiert, die Petrolätherfraktionen werden vereinigt und am Rotavapor bei Raumtemperatur eingedampft. Das so erhaltene Oel ist das 5,7,3',4'-tetra-O-Methoxymethyl-3-O-methyl-(+)-cyanidan-3-ol. IR (nujol) $\nu$ ($cm^{-1}$): 2920, 1615, 1590, 1507, 1494, 1440, 1400, 1260, 1220, 1155, 1070, 1000, 920, 822.

Beispiel 58

Man gibt in einen 1 Liter Kolben 110 g Kaliumcarbonat unter schwachem Stickstoff, dann 250 ml Dimethylformamid, und giesst eine Lösung von 29,0 g (+)-Cyanidan-3-ol in 250 ml getrocknetem Dimethylformamid und 71,25 ml Benzylbromid gelöst in 100 ml Dimethylformamid. Man erwärmt auf 100°C während 5 1/2 Stunden unter Rühren und im Stickstoffstrom. Die erhaltene Suspension wird heiss filtriert, das Filtrat bei 80°C im Vakuum am Rotavapor eingedampft und der Rückstand mit 250 ml Chloroform gelöst. Die chloroformlösung wird filtriert, 3 Mal mit 250 ml Wasser gewaschen, über Magnesiumsulfat getrocknet und zur Trockne eingedampft. Man nimmt dem Rückstand in 750 ml am Rückfluss kochenden Tetrachlorkohlenstoff auf, trennt den braunen klebrigen Rückstand ab und lässt bei Raumtemperatur unter Rühren und gelegentlichem Abkühlen im Eisschrank kristallieren. Das erhaltene Produkt ist identisch mit dem des Beispiels 37. F = 144—145°C.

Beispiel 59

Man stellt ausgehend von 5 g Palladiumchlorid, wie in Beispiel 1 beschrieben Palladiumschwarz her, gibt dazu eine Lösung von 40 g 5,7,3',4'-tetra-O-Benzyl-3-O-phenylacetyl-(+)-cyanidan-3-ol in 2,25 Liter Aethylacetat, hydriert während 3 Tagen, wobei 1,25 Liter Wasserstoff verbraucht werden, filtriert vom Palladium ab, wäscht dieses 2 Mal mit 300 ml Aethylacetat und engt die vereinigten Acetatlösungen unter vermindertem Druck ein. Zum Rückstand gibt man 500 ml Aethanol, dampft die Lösung auf ein Volumen von ungefähr 50 ml ein und wiederholt diese Operation. Man gibt dann 250 ml Aethanol und 250 ml Wasser zu, und dampft ein auf ein Volumen von ca. 50 ml. Man gibt 500 ml Wasser zu, dampft sie wiederum auf etwa 50 ml in Vakuum ein, fügt 500 ml Wasser zu und dampft erneut unter vermindertem Druck ein. Der Rückstand wird über Nacht zur Gewichtskonstanz getrocknet. Das so erhaltene 3-O-Phenyl-acetyl-(+)-cyanidan-3-ol schmilzt bei 102—103°C.

Beispiel 60

In einen 250 ml Kolben, gibt man eine Lösung von 24,8 g Nikotinoylchlorid und 100 ml wasserfreiem Pyridin, fügt dann tropfenweise unter Rühren und Stickstoff 56 g 5,7,3',4'-tetra-O-Benzyl-(+)-cyanidan-3-ol in 4 Portionen zu (1 zu 26 g und 3 zu 10 g, wobei zwischen jedem Zufügen eine Stunde liegt) und wärmt während 20 Stunden auf 60°C. Nach dem Abkühlen giesst man die Lösung auf 1 kg gestossenes Eis und rührt bis dass das Eis geschmolzen ist. Man dekantiert vom klebrigen Niederschlag ab, wäscht diesen 2 Mal mit Wasser, 2 Mal 1N Natriumbicarbonat, dann 2 Mal mit Wasser. Das Unlösliche das immer noch klebrig ist, wird über Nacht im Vakuum getrocknet, man gibt 120 ml Aceton zu, rührt während 10 Minuten und lässt 2 Stunden bei —30°C stehen. Der Niederschlag wird abfiltriert und erneut mit Aceton behandelt. Das 5,7,3',4'-tetra-O-Benzyl-3-O-nikotinoyl-(+)-cyanidan-3-ol schmilzt nach dem Umkristallisieren aus 950 ml Methoxyäthanol und 1,9 Liter wasserfreiem Aethanol, bei 137 bis 138°C.

Beispiel 61

Man hydriert 1,5 g 5,7,3',4'-tetra-O-Benzyl-3-O-nikotinoyl-(+)-cyanidan-3-ol in Gegenwart von

100 ml wasserfreiem Aethanol, 300 mg Trifluormethansulfonsäure und 1,0 g Palladium auf Aktivkohle (10%) in dem man auf 50°C erwärmt. Die Reaktion dauert 5 Stunden und verbracht 500 ml Wasserstoff. Die Mischung wird abgekühlt, dann filtriert und das Filtrat zur Trockne eingedampft. Zum Rückstand gibt man 100 ml destilliertes Wasser, dampft dann auf ca. 10 ml ein, wiederholt diese Operation 3 Mal und lyophilisiert das Produkt. Man erhält so das Trifluormethansulfonat des 3-O-(3-piperidincarbonyl)-(+)-cyanidan-3-ol vom F = 125—130°C. (Mischung von 2 Diastereomeren).

Beispiel 62

In einen 500 ml Kolben der mit einem Rückflusskühler und einem Vibromischer ausgerüstet ist, gibt man 5,2 g 5,7,3',4'-tetra-O-Benzyl-(+)-cyanidan-3-ol und 200 ml Toluol. Man erwärmt auf ca. 90°C bis zur Lösung, gibt dann 200 ml einer 50%igen Lösung von Natriumhydroxyd, 2,7 g Tetrabutylammoniumhydrogensulfat und 3,0 g 2-Cyclohexyl-äthylbromid zu. Man hält unter gutem Rühren während 10 Stunden bei 90°C, gibt dann 1,5 g 2-Cyclohexyl-äthylbromid zu und rührt weitere 15 Stunden bei 90°C, dann kühlt das Reaktionsgemisch auf Raumtemperatur ab, trennt die beiden Phasen, wäscht die organischen Phasen 3 Mal mit 250 ml Wasser und trocknet das Toluol über Magnesiumsulfat. Man dampft ein, nimmt den Rückstand in 60 ml abs. Aethanol bei 60°C auf, rührt während 15 Minuten, filtriert heiss. Der unlösliche Anteil wird mit Vakuum getrocknet und aus 80 ml Methoxyäthanol umkristallisiert. Man erhält so das 5,7,3',4'-tetra-O-Benzyl-3-O-(2-cyclohexyl-äthyl)-(+)-cyanidan-3-ol vom F = 110°C.

Beispiel 63

Man hydriert 1,0 g 5,7,3',4'-tetra-O-Benzyl-3-O-(2-cyclohexyl-äthyl)-(+)-cyanidan-3-ol in 90 ml Methylacetat in Gegenwart von 400 mg Palladium (10%) auf Aktivkohle. 180 ml Wasserstoff werden verbraucht. Man filtriert, dampft das Lösungsmittel ab, löst den Rückstand in 100 ml Aethanol, dampft beinahe zur Trockne, gibt 100 ml Aethanol zu, das dann erneut verdampft. Man fügt 50 ml Aethanol und 50 ml Wasser zu und dampft die Mischung ein. Der Rückstand wird in 150 ml Wasser aufgenommen, zur Trockne eingedampft und der Rückstand über Phosphorpentoxyd im Hochvakuum zur Gewichtskonstanz getrocknet. Man erhält so das 3-O-(2-Cyclohexyl-äthyl)-(+)-cyanidan-3-ol vom F = 99—102°C.

Beispiel 64

In einen 6 Liter Kolben mit einem Rückflusskühler und einem mechanischen Rührer gibt man 65 g 5,7,3',4'-tetra-O-Benzyl-(+)-cyanidan-3-ol und 2 Liter Toluol. Man erwärmt bis zur Lösung, gibt dann 1,5 Liter einer 50%igen Natronhydroxydlösung, 34 g Tetrabutylammoniumhydrogensulfat und 40 g 3-Phenyl-propylbromid. Man erwärmt auf 50°C unter gutem Rühren auf 40 Stunden, trennt nach dem Abkühlen auf Raumtemperatur die beiden Phasen und wäscht die organische Phase 3 Mal mit einem Liter Wasser. Man trocknet das Toluol über Magnesiumsulfat und dampft ein. Der ölige Rückstand wird mit 200 ml Petroläther, dann mit 150 ml wasserfreiem Aethanol gewaschen und das Produkt aus 600 ml Methoxyäthanol umkristallisiert. Man erhält das 5,7,3',4'-tetra-O-Benzyl-3-O-(3-Phenyl-propyl)-(+)-cyanidan-3-ol vom F = 96—97°C.

Beispiel 65

Man hydriert 40,0 g 5,7,3',4'-tetra-O-Benzyl-3-O-(3-phenylpropyl)-(+)-cyanidan-3-ol gelöst in 3 Liter Aethylacetat in Gegenwart von 16 g Palladiumkohle (10%). Die Reaktion dauert 6 Stunden und verbraucht 6,2 Liter Wasserstoff. Man filtriert, dampft das Lösungsmittel ab, gibt 250 ml Wasser zu, schüttelt, dampft unter Vakuum ein bis zu einer starken Ausfällung. Man filtriert, wäscht den Niederschlag gut mit Wasser und erhält so das 3-O-(3-phenyl-propyl)-(+)-cyanidan-3-ol das bei 190—192°C schmilzt.

Beispiel 66

In einem 3 Liter Kolben der einen Rückflusskühler und einen mechanischen Rührer besitzt, gibt man 52 g 5,7,3',4'-tetra-O-Benzyl-(+)-cyanidan-3-ol und 500 ml Toluol. Man erwärmt bis zur Lösung, gibt 187 g Propylchlorid, 750 ml Natriumhydroxyd (50%ig) und 6,8 g Tetrabutylammoniumhydrogensulfat zu. Man wärmt auf 50°C, rührt gut während 24 Stunden und lässt dann auf Raumtemperatur abkühlen. Die beiden Phasen werden getrennt, die organische Phase 3 Mal mit 500 ml einer gesättigten wässrigen Kochsalzlösung gewaschen, über Magnesiumsulfat getrocknet und eingedampft. Zum Rückstand gibt man 100 ml Tetrachlorkohlenstoff und dampft zur Trockne. Das erhaltene 5,7,3',4'-tetra-O-Benzyl-3-O-propyl-(+)-cyanidan-3-ol schmilzt nach dem Umkristallisieren aus einer Mischung von 1,8 Liter Aethanol und 0,3 Liter Aceton bei 92—93°C.

Beispiel 67

Wie in Beispiel 1 stellt man aus 12 g Palladiumchlorid Palladiumscharz her, gibt eine Lösung von 41,6 g 5,7,3',4'-tetra-O-Benzyl-3-O-propyl-(+)-cyanidan-3-ol in 1,8 Liter Athylacetat zu. Man hydriert während 6 Stunden, 6,25 Liter Wasserstoff werden verbraucht. Man filtriert, wäscht das Palladium 2 Mal mit 200 ml Aethylacetat, dampft die vereinigten Lösungen ein, gibt 500 ml Wasser zum Rückstand, und dampft ihn unter vermindertem Druck auf ein Volumen von ungefähr 50 ml ein. Man wiederholt diese Operation 3 Mal, dann wird lyophilisiert. Man erhält so das 3-O-Propyl-(+)-cyanidan-3-ol vom F = 94—96°C.

Beispiel 68

In einen 1 Liter Kolben der mit einem Rückflusskühler, der ein Kalziumchloridrohr trägt, einem Einfüllstutzen mit Druckkompensation, einem Rührwerk und einem Stickstoffeinlass ausgerüstet ist, gibt man eine Lösung von 65,0 g 5,7,3',4'-tetra-O-Benzyl-(+)-cyanidan-3-ol in 500 ml wasserfreiem Pyridin. Dazu fügt man, tropfenweise unter Stickstoff, 34,5 g Diäthylchlorophosphat und rührt bei Raumtemperatur unter Stickstoff während 8 Stunden. Die Reaktionsmischung wird dann auf 1 Liter Eiswasser gegossen und gut durchgemischt. Man dekantiert das Wasser von der klebrigen Masse ab, wäscht Letztere 5 Mal mit 500 ml Wasser, und lässt eine Nacht bei 40°C unter Vakuum stehen. Das erhaltene Produkt wird aus 720 ml Tetrachlorkohlenstoff und 2,8 Petroläther umkristallisiert. Man erhält so das 2R, 3S-5,7,3',4'-tetra-Benzyloxy-3-flavanyl-diäthyl-phosphat, das bei 96°C schmilzt.

Beispiel 69

Ausgehend von 13 g Palladiumchlorid stellt man wie im Beispiel 1 beschrieben Palladiumschwarz her, gibt eine Lösung von 30,0 g 2R, 3S-5,7,3',4'-tetra-Benzyloxy-3-flavanyl-diäthyl-phosphat in 1,3 Liter Aethylacetat zu, hydriert während 3 Stunden wobei 3,9 Liter Wasserstoff verbraucht werden. Man filtriert das Palladium ab, wäscht es 2 Mal mit 200 ml Aethylacetat, dampft die vereinigten Lösungen ein, gibt 750 ml Wasser zum Rückstand und dampft erneut unter vermindertem Druck auf ein Volumen auf ungefähr 100 ml ein. Man wiederholt dies 2 Mal, und lyophilisiert. Man erhält so das 2R, 3S-5,7,3',4'-tetra-Hydroxy-3-flavanyl vom F = 121—123°C.

Beispiel 70

In einen 500 ml Kolben mit einem Abflusskühler und einem Mechanischen Rührer gibt man 1,3 g 5,7,3',4'-tetra-O-Benzyl-(+)-cyanidan-3-ol, 100 ml Toluol, 100 ml einer wässrigen 50%igen Lösung von Natriumhydroxyd, 500 mg Dimethylsulfat und 68 mg Tetrabutylammoniumhydrogensulfat. Man erwärmt die Mischung auf 50°C während einer Stunde und rührt stark, lässt dann auf Raumtemperatur abkühlen und trennt die beiden Phasen. Die organische Phase wird 3 Mal mit 100 ml Wasser gewaschen, über Magnesiumsulfat getrocknet und eingedampft. Man kristallisiert das erhaltene 5,7,3',4'-tetra-O-Benzyl-3-O-methyl-(+)-cyanidan-3-ol aus 13 ml Methoxyäthanol. F = 124—125°C.

Beispiel 71

In einen 250 ml Kolben gibt man 500 mg Palladiumschwarz, 120 ml Aethanol, 650 mg 5,7,3',4'-tetra-O-Benzyl-(+)-cyanidan-3-ol und 60 ml Cyclohexen. Unter Rühren kocht man das Reaktionsgemisch 4 Stunden am Rückfluss, filtriert das Palladium bei Raumtemperatur ab und destilliert das Filtrat unter vermindertem Druck. Das zurückbleibende (+)-Cyanidan-3-ol schmilzt nach dem Umkristallisieren aus Wasser bei 207—210°C.

Beispiel 72

Ein 500 ml Kolben wird mit einem mehcanischen Rührer, einen Zuflussstutzer, einem Kalziumchloridrohr und einer Zuführung für Stickstoff ausgerüstet. Das Ganze wird getrocknet und und unter Stickstoff gesetzt bevor man 100 ml wasserfreies Dimethylformamid zugibt. Man kühlt auf —3°C, gibt 9,28 g einer öligen Dispersion von Natriumhydrid (55%) und dann unter starkem Rühren eine Lösung von 14,5 (+)-Cyanidan-3-ol in 200 ml wasserfreiem Dimethylformamid, 1,698 g Tetra-n-butylammoniumhydrogensulfat zu und hält unter Rühren 1 Stunde bei 0°C. Man gibt dann innerhalb 15 Minuten eine Lösung von 25,9 ml Benzylchlorid in 25 ml wasserfreiem Dimethylformamid zu und rührt während 30 Minuten bei 0°C. Man lässt die Temperatur auf Raumtemperatur steigen, hält das Reaktionsgemisch während 24 Stunden unter starkem Rühren, filtriert und dampft unter vermindertem Druck ein. Der Rückstand wird in 250 ml Chloroform aufgenommen, die Lösung 4 Mal mit 250 ml destilliertem Wasser gewaschen, über Magnesiumsulfat getrocknet, filtriert und eingedampft. Der Rückstand wird mit 50 ml Hexan gewaschen und dann auf Gewichtskonstanz gebracht. Diese Masse wird heiss in 500 ml Tetrachlorkohlenstoff gelöst, heiss filtriert und durch Abkühlen auf Raumtemperatur und anschliessendem Einengen auf 350 ml und 150 ml das 5,7,3',4'-tetra-O-Benzyl-(+)-cyanidan-3-ol das mit dem im Beispiel 37 erhaltenen Produkt identisch ist.

Beispiel 73

Wie in Beispiel 58 gibt man nach der Einführung der Cyanidanollösung 13,22 g 18-Crown-6 und ersetzt das Benzylbromid durch 69 ml Benzylchlorid. Man erwärmt dann während 20 Stunden unter starkem Rühren auf 60°C. Man erhält so das 5,7,3',4'-tetra-O-Benzyl-(+)-cyanidan-3-ol das mit dem im Beispiel 37 erhaltenen Produkt identisch ist.

Beispiel 74

Man verfährt analog Beispiel 73, ersetzt jedoch das 18-Crown-6 durch 16,98 g Tetra-n-butylammoniumhydrogensulfat. Man erhält 44,5 g 5,7,3',4'-tetra-O-Benzyl-(+)-cyanidan-3-ol.

Beispiel 75

In einen 5 Liter Kolben mit einem Rückflusskühler und einem mechanischen Rührer gibt man 130 g 5,7,3',4'-tetra-O-Benzyl-(+)-cyanidan-3-ol, 250 g 1-Heptanchlorid und 25 g Tetrabutylammoniumhydrogensulfat. Man fügt, 3,75 Liter einer Lösung von Natriumhydroxyd (50%) zu, erwärmt auf 50°C während 6 Stunden, lässt auf Raumtemperatur abkühlen, gibt 1,25 Liter Dichlormethan zu und trennt die beiden Phasen. Die organische Phase wird 3 Mal mit 750 ml Wasser gewaschen, über Magnesiumsulfat getrocknet, das Lösungsmittel eingedampft, dann 4 Liter Petroläther zugegeben und die Mischung während der Nacht gerührt. Man kühlt während einer halben Stunde auf —20°C, filtriert den Niederschlag ab, wäscht ihn mit 500 ml Petroläther und kristallisiert ihn 2 Mal in einer Mischung von 1,5 Liter Aethanol und 400 ml Aethylacetat. Man erhält so das 5,7,3',4'-tetra-O-Benzyl-O-heptyl-(+)-cyanidan-3-ol vom F = 65°C.

Beispiel 76

In einer 3 Liter Kolben mit einem Rückflusskühler und einem mechanischen Rührer gibt man 52 g 5,7,3',4'-tetra-O-Benzyl-(+)-cyanidan-3-ol und 650 ml Toluol. Man kocht bis zur Lösung, gibt dann 115 g 1-Chlor-dodecan, 27 g Tetrabutylammoniumhydrogensulfat und 750 ml einer 50%igen Natriumhydroxydlösung zu. Man erwärmt die Mischung während 4 Tagen auf 50°C unter starkem Rühren, kühlt ab auf Raumtemperatur, trennt die beiden Phasen und wäscht die organische Phase 3 Mal mit 500 ml Wasser. Man trocknet über Magnesiumsulfat, dampft das Lösungsmittel ein und mischt 2 Mal den Rückstand mit 500 ml Petroläther. Nach dem Filtrieren und dem Trocknen kristallisiert man das erhaltene Produkt aus 800 ml Aceton und 3,2 Liter Aethanol. Man erhält so das 5,7,3',4'-tetra-O-Benzyl-3-O-dodecyl-(+)-cyanidan-3-ol vom F = 98—99°C.

Beispiel 77

In einen 1 Liter Kolben mit einem Kühler und einem mechanischem Rührer gibt man eine Mischung von 26 g 5,7,3',4'-tetra-O-Benzyl-(+)-cyanidan-3-ol, 73 g 1-Chlor-hexadecan und 5 g Tetrabutylammoniumhydrogensulfat. Man fügt 750 ml einer wässrigen Natriumhydroxydlösung (50%) zu, erwämt auf 50°C unter starkem Rühren während 24 Stunden. Nach dem Abkühlen auf Raumtemperatur gibt man 250 ml Dichlormethan zu, trennt die beiden Phasen und wäscht die organische Phase mit 250 ml Wasser. Man dampft das Lösungsmittel ab, gibt 350 ml Aethanol zu und rührt während 2 Stunden. Man kühlt auf —10°C ab, filtriert, wäscht den Rückstand mit 50 ml Aethanol und trocknet im Vakuum. Das erhaltene 5,7,3',4'-tetra-O-Benzyl-3-O-hexadecyl-(+)-cyanidan-3-ol schmilzt nach dem Umkristallisieren aus 300 ml Aethanol und 70 ml Aethylacetat bei 88—89°C.

Beispiel 78

In einem 3 Liter Kolben mit einem mechanischen Rührer und einem Rückflusskühler gibt man 52 g 5,7,3',4'-tetra-O-Benzyl-(+)-cyanidan-3-ol, 162 g 1-Chlor-octadecan und 10 g Tetrabutylammoniumhydrogensulfat. Man gibt 1,5 Liter einer 50%igen Lösung von Natriumhydroxyd zu, erwärmt während 28 Stunden auf 50°C unter gutem Rühren, gibt nach dem Abkühlen auf Raumtemperatur 500 ml Dichlormethan zu und trennt die beiden Phasen. Man wäscht die organische Phase mit 500 ml Wasser, wobei sich eine Emulsion bildet. Man trennt die beiden Phasen durch Filtrieren über einem Papier zur Trennung verwendet werden kann, trocknet die organische Phase über Magnesiumsulfat und dampft ein. Man fügt 750 ml Aethanol zu, rührt während 2 Stunden, filtriert und wäscht den Rückstand mit 100 ml Aethanol. Das erhaltene 5,7,3',4'-tetra-O-Benzyl-3-O-octadecyl-(+)-cyanidan-3-ol schmilzt nach dem Umkristallisieren aus 600 ml Aethanol und 260 ml Aethylacetat (2 Mal) bei 86°C.

Beispiel 79

In einem 5-Liter Kolben hydriert man 56 g 5,7,3',4'-tetra-O-Benzyl-3-O-heptyl-(+)-cyanidan-3-ol gelöst in 2,25 Liter Aethylacetat, in Gegenwart von 40 g Palladium auf Kohle (10%), filtriert den Katalysator ab und wäscht ihn 2 Mal mit 300 ml Aethylacetat. Die vereinigten Lösungen werden eingedampft, der Rückstand mit 250 ml Wasser gut gerührt, das Wasser dann eingedampft bis auf ein Volumen von 50 ml. Man wiederholt dies 2 Mal und dampft dann unter starkem Vakuum über Phosphorpentoxyd ein. Man erhält so das 3-O-Heptyl-(+)-cyanidan-3-ol vom F = 157—159°C.

Beispiel 80

In einem 6 Liter Kolben hydriert man 40 g 5,7,3',4'-tetra-O-Benzyl-3-O-dodecyl-(+)-cyanidan-3-ol gelöst in 3 Liter Aethylacetat in Gegenwart von 20 g Palladium auf Kohle (10%). Die Reaktion dauert 6 Stunden und verbraucht 6,2 Liter Wasserstoff. Man filtriert vom Katalysator ab, wäscht ihn 2 Mal mit 300 ml Aethylacetat, dampft die vereinigten Lösungen zur Trockne ein und nimmt den öligen Rückstand in 1 Liter Aethanol auf. Man engt auf ein Volumen von 50 ml ein, gibt einen Liter Wasser zu und engt erneut auf 50 ml ein, dann gibt man 1 Liter Wasser zu, engt auf ein Volumen von 100 ml ein, filtriert und wäscht den Rückstand mit Wasser. Nach dem Trocknen im Hochvakuum über Phosphorpentoxy erhält man das 3-O-dodecyl-(+)-cyanidan-3-ol vom F = 158—160°C.

Beispiel 81

In einem 3 Liter Kolben hydriert man 26,3 g 5,7,3',4'-tetra-O-Benzyl-3-O-hexadecyl-(+)-cyanidan-3-ol gelöst in 15, Liter Aethylacetat in Gegenwart von 16 g Palladium auf Kohle (10%), filtriert vom katalysator ab und wäscht ihn 2 Mal mit 100 ml Aethylacetat. Die vereinigten Lösungen werden eingedampft, der Rückstand in 100 ml Aether aufgenommen und während einer Stunde mit 5 g Aktivkohle behandelt. Man filtriert, dampft das Filtrat ein, rührt gut mit 60 ml Wasser und dampft auf ein Volumen von 10 ml ein. Dies wird 2 Mal wiederholt, dann dampft man zur Trockne ein und trocknet den Rückstand im Hochvakuum über Phosphorpentoxyd zur Gewichtskonstanz. Man erhält so das 3-O-Hexadecyl-(+)-cyanidan-3-ol vom F = 152—154°C.

Beispiel 82

In einem 8 Liter Kolben hydriert man 83,7 g 5,7,3',4'-tetra-O-Benzyl-3-O-octadecyl-(+)-cyanidan-3-ol gelöst in 4,5 Liter Aethylacetat in Gegenwart von 48 g Palladium auf Kohle (10%). Man filtriert vom Katalysator ab, wäscht diesen 2 Mal mit 400 ml Aethylacetat, dampft die vereinigten Lösungen ein und kristallisiert den Rückstand aus 260 ml Chloroform, das 4% Methanol enthält, um. Der feste Rückstand wird mit 100 ml Wasser behandelt, dann auf ein Volumen von ungefähr 25 ml eingedampft. Man wiederholt dies 2 Mal, dampft am Schluss zur Trockne ein und trocknet im Hochvakuum über Phosphorpentoxyd zur Gewichtskonstanz. Man erhält so das 3-O-Octadecyl-(+)-cyanidan-3-ol vom F = 162—164°C.

Beispiel 83

In einen 750 ml Kolben mit einem Rückflusskühler und einem mechanischen Rührer gibt man eine Lösung von 52 g 5,7,3',4'-tetra-O-Benzyl-(+)-cyanidan-3-ol in 300 ml Dichlormethan, 150 ml einer wässrigen 50%igen Natronlauge und 6,8 g Tetrabutylammoniumhydrogensulfat. Man kocht die Mischung am Rückfluss während 24 Stunden unter gutem Rühren, kühlt dann auf Raumtemperatur ab und trennnt die beiden Phasen. Die organische Phase wird 3 Mal mit 300 ml Wasser gewaschen, dann über Magnesiumsulfat getrocknet. Man dampft das Lösungsmittel ab und kristallisiert das erhaltene Di-(5,7,3',4'-tetra-O-Benzyl-(+)-cyanidanyl-3-oxy)-methan aus einer Mischung von 2,4 Liter Aethanol und 1,2 Liter Aceton um. F = 103—104°C.

Beispiel 84

Man bereitet, wie in Beispiel 1 beschrieben, Palladium-schwarz ausgehend von 5 g Palladiumchlorid, gibt eine Lösung von 31,0 g Di-(5,7,3',4'-tetra-O-Benzyl-(+)-cyanidanyl-3-oxy)-methan in 700 ml Aethylacetat zu. Man hydriert während 3 Stunden, wobei 4,8 Liter Wasserstoff verbraucht werden. Man filtriert vom Palladium ab, wäscht dieses 2 Mal mit 100 ml Aethylacetat, und dampft die verinigten Lösungen ein. Zum Rückstand gibt man 1 Liter Wasser das mit einigen Tropfen Pyridin auf pH 7 eingestellt wurde, konzentriert auf ca. 100 ml unter vermindertem Druck, wiederholt dies 2 Mal, gibt dann 900 ml reines Wasser zu und dampft auf ein Volumen von 200 ml ein. Man lyophilisiert und erhält das Di-(cyanidan-3-yl-oxy)-methan vom F = 162—165°C.

Beispiel 85

In analoger Weise zu den in dem vorstehend bescchriebenen Beispiel erhält man: das 3,5,7,3',4'-penta-O-Methoxymethyl-(+)-cyanidan-3-ol,
das 3,5,7,3',4'-penta-O-Allyl-(+)-cyanidan-3-ol,
das 5,7,3',4'-tetra-O-Trimethylsilyl-(+)-cyanidan-3-ol,
das 5,7,3',4'-tetra-O-Benzyl-3-O-(3-N,N-dimethylamino-propyl)-(+)-cyanidan-3-ol,
das 3-O-(3-N,N-dimethylamino-propyl)-(+)-cyanidan-3-ol in Form seines Hydrochlorids,
das 5,7,3',4'-tetra-O-Benzyl-3-O-4,4-äthylendioxy-p-fluorphenyl-butyl)-(+)-cyanidan-3-ol,
das 3-O-(4,4-äthylendioxyd-4-p-fluorphenyl-butyl)-(+)-cyanidan-3-ol,
das 5,7,3',4'-tetra-O-Benzyl-3-O-(2,3-dihydroxy-propyl)-(+)-cyanidan-3-ol und
das 3-O-(2,3-dihydroxy-propyl)-(+)-cyanidan-3-ol.

**Patentansprüche**

1. Neue O-substutierte Derivate des (+)-Cyanidan-3-ols der Formel I

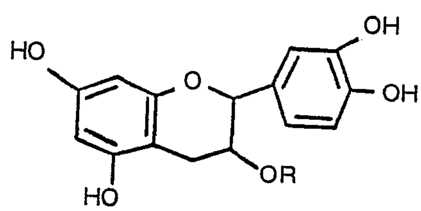

(I)

worin R

einen unsubstituierten oder durch Hydroxy, Niederalkanoyloxy, Niederalkyloxy, Niederalkanoyl, Cyano, Amino, Mono- oder Diniederalkyl-amino, Niederalkylenamino, Oxa- oder Azaniederalkylenamino, Carbamoyl, Mono- oder Diniederalkyl-carbamoyl, oder Halogenatome susbtituierten Alkyl- oder Alkenyl-Rest,

einen unsubstituierten oder durch eines oder mehrere Hydroxy-, Niederalkoxy, Niederalkyl, Carboxy, Oxo, Niederalkanoyl, Amino, Mono- oder Di-alkyl-amino oder Carboniederalkoxy substituierten Cycloalkyl-Rest mit 3 bis 6 Ringkohlenstoffatomen,

ein Cycloalkyl-niederalkyl-Rest, der im Ring 3 bis 6 Kohlenstoffatome aufweist und unsubstituiert oder durch mondestens einen Niederalkylrest substituiert sein kann,

eine Phenyl- oder Naphthyl-Rest, der durch Hydroxy, Niederalkoxy, Carboxy, Carboniederalkoxy, Nitro, Amino-, Mono- oder Diniederalkylamino, oder Halogenatome, mono-, di- oder trisubstituiert sein kann,

ein Phenyl- oder Naphthyl-niederalkyl- oder -niederalkenyl-Rest der im aromatischen Kern durch Hydroxy, Niederalkoxy, Carboxy, Carboniederalkoxy, Alkanoyl, Nitro, Amino, Mono- oder Dialkyl-amino, Di-niederalkyl-carbamoyl oder Halogenatome, mono- di- oder trisubstituiert sein kann (ausgenommen Trihydroxy),

ein gesättigter oder ungesättigter Aza-, Thia-, Oxa-, Thiaza-, Oxaza- oder Diaza-cyclischer Rest, der an einen Phenylkern ankondensiert sien kann, und der 2—7 Kohlenstoffatome im heterocyclischen Ring aufweist und durch Alkyl, Hydroxy, Niederalkoxy, Nitro, Carboxy, Carbo-niederalkoxy oder Alkanoyloxy mono-, di oder polysubstituiert sein kann,

ein Heterocyclyl-niederalkyl-Rest, dessen Heterocyclus ein gesättigter oder ungesättigter Aza-, Thia-, Oxa-, Thiaza-, Oxaza- oder Diaza-cyclus darstellt,

ein Rest einer Alkancarbonsäure mit mindestens 3 Kohlenstoffatomen, Niederalkencarbonsäure, Niederalkandicarbonsäure, oder !Niederalkendicarbonsäure,

ein unsubstituierter oder durch eine oder mehrere Hydroxy, Niederalkyloxy, Carboxy, Carboniederalkoxy, Niederalkyl, Niederalkanoyl, Oxo oder Halogen substituierter Cycloalkancarbonsäure-Rest,

Benzoyl oder Naphthoylrest, der gegebenenfalls durch ein Hydroxy, Niederalkoxy, Alkanoyloxy, Carboxy, Carboniederalkoxy, Nitro, Amino oder Halogen substituiert sein kann,

ein Phenyl- oder Naphthyl-niederalkanoyl-Rest, der im aromatischen Teil unsubstituiert oder durch Hydroxy, Niederalkoxy, Carboxy, Carboniederalkoxy, Niederalkanoyl, Niederalkanoyloxy, Nitro, Amino, Mono oder Diniederalkylamino oder Halogen substituiert sein kann,

ein Rest einer Aza-, Thia-, Oxa-, Thiaza-, Oxaza oder Diaza-cyclischen Säure, die gesättigt oder ungesättigt, oder auch an einen Phenylrest ankondensiert sein kann,

ein Alkoxycarbonyl-Rest,

ein Phenyloxycarbonyl-Rest,

ein Carbamoyl-Rest, der gegebenenfalls mono- oder disubstituiert sein kann durch Niederalkylreste, Phenyl- oder benzylreste, die gegebenenfalls im Ring durch Hydroxy, Niederalkoxy, Niederalkanoyloxy, oder Halogenatome substituiert sein können,

ein Rest einer Niederalkyl-sulfonsäure, oder Phenylsulfonsäure, die im Ring durch Niederalkyl, Niederalkoxy oder Halogenatome substituiert sein kann, oder

den Rest einer unsubstituierten oder durch 1 oder 2 Niederalkyl- oder 1 oder 2 Phenylreste veresterten Phosphorsäure sein kann, wobei Nieder einen Rest mit bis 7 C-Atomen bedeutet, und ihre Salze.

2. 3-O-Methyl-(+)-cyanidan-3-ol
3. 3-O-Butyl-(+)-cyanidan-3-ol
4. 3-O-Butyryl-(+)-cyanidan-3-ol
5. 3-O-(3,3-Dimethyl-butanoyl)-(+)-cyanidan-3-ol
6. 3-O-(3-carboxy-propionyl)-(+)-cyanidan-3-ol
7. 3-O-Decanoyl-(+)-cyanidan-3-ol
8. 3-O-Dodecyl-(+)-cyanidan-3-ol
9. 3-O-Heptyl-(+)-cyanidan-3-ol
10. 3-O-Octadecyl-(+)-cyanidan-3-ol
11. 3-O-Palmitoyl-(+)-cyanidan-3-ol
12. 3-O-(2-Carboxy-cyclohexan-carbonyl)-(+)-cyanidan-3-ol
13. 3-O-benzoyl-(+)-cyanidan-3-ol
14. 3-O-(4-Fluor-benzoyl)-(+)-cyanidan-3-ol
15. 3-O-Protocatechyl-(+)-cyanidan-3-ol
16. 3-O-Acetylsalicylyl-(+)-cyanidan-3-ol
17. 3-O-(2-Carboxy-benzoyl-(+)-cyanidan-3-ol
18. 3-O-(N-phenylcarbamoyl)-(+)-cyanidan-3-ol
19. 3-O-Methansulfonyl-(+)-cyanidan-3-ol
20. 3-O-(4-Methyl-benzolsulfonyl)-(+)-cyanidan-3-ol
21. 3-O-((2-Cyano-äthyl)-(+)-cyanidan-3-ol
22. 3-O-(3-Amino-propyl)-(+)-cyanidan-3-ol

23. 3-O-Methoxymethyl-(+)-cyanidan-3-ol

24. 3-O-Aethoxycarbonyl-(+)-cyanidan-3-ol

25. Pharmazeutische Zusammensetzungen, gekennzeichnet durch einen Gehalt an einer Verbindung gemäss Anspruch 1 oder einem pharmazeutisch verwendbaren Salz derselben und mindestens einem pharmazeutischen Trägerstoff.

26. Pharmazeutische Zusammensetzungen, gekennzeichnet durch ein Gehalt an einer Verbindung gemäss einem der Ansprüche 2—24 oder einem pharmazeutisch verwendbaren Salz derselben, und mindestens einen pharmazeutischen Trägerstoff.

27. Verwendung einer Verbindung gemäss Anspruch 1 als Wirkstoff zur Herstellung eines Arzneimittels zur Behandlung von Leberkrankheiten.

28. Verwendung einer Verbindung gemäss einem der Ansprüche 2—24 als Wirkstoff zur Herstellung eines Arzneimittels zur Behandlung von Leberkrankheiten.

29. Verfahren zur Herstellung von O-substituierten Derivaten des (+)-Cyanidan-3-ols der Formel I

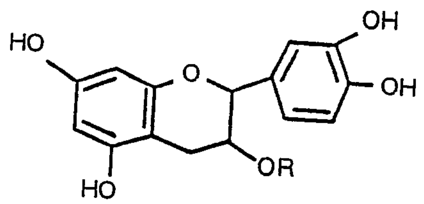

(I)

worin R die in Anspruch 1 angegebene Bedeutung hat und ihren Salzen, dadurch gekennzeichnet, dass man in einer Verbindung der Formel II

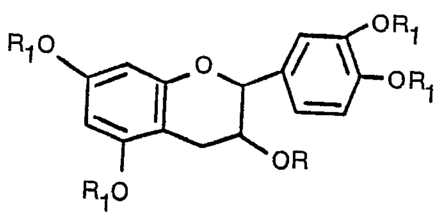

(II)

worin $R_1O$ eine geschützte Oxygruppe, die leicht hydrolisierbar oder hydrogenolysierbar ist, darstellt und R die obengenannte Bedeutung besitzt, den Rest $OR_1$ zur Hydroxygruppe aufspaltet und wenn erwünscht in einer erhaltenen Verbindung einen Rest R durch Reduktion umwandelt, und/oder, dass man ein erhaltenes Salz in die freie Verbindung oder eine freie Verbindung mit salzbildenden Gruppen in das Salz unwandelt.

## Claims

1. A novel O-substituted derivative of (+)-cyanidan-3-ol of the formula I

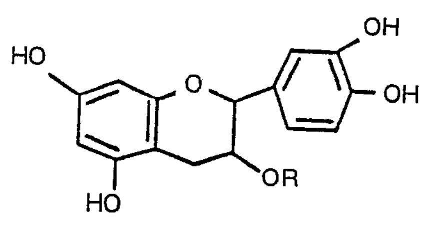

(I)

wherein R is

— an alkyl or alkenyl radical which is unsubstituted or substituted by hydroxy, lower alkanoyloxy, lower alkoxy, lower alkanoyl, cyano, amino, mono- or di-lower alkylamino, lower alkyleneamino, oxa- or aza-lower alkyleneamino, carbamoyl, mono- or di-lower alkylcarbamoyl or by halogen atoms;

— a cycloalkyl radical containing from 3 to 6 ring carbon atoms which is unsubstituted or substituted by one or more hydroxy, lower alkoxy, lower alkyl, carboxy, oxo, lower alkanoyl amino, mono- or di-lower alkylamino or lower alkoxycarbonyl groups;

— a cycloalkyl-lower alkyl radical containing from 3 to 6 ring carbon atoms which is unsubstituted or substituted by at least one lower alkyl group;

— a phenyl or naphthyl radical which may be substituted by one, two or three hydroxy, lower alkoxy, carboxy, lower alkoxycarbonyl, nitro, amino or mono- or di-lower alkylamino groups, or by one, two or three halogen atoms;

— a phenyl-lower alkyl or naphthyl-lower alkyl radical or phenyl-lower alkenyl or naphthyl-lower alkenyl radical which may be substituted in the aromatic nucleus by one, two or three hydroxy, lower alkoxy, carboxy, lower alkoxycarbonyl, lower alkanoyl, nitro, amino, mono- or di-lower alkylamino, di-lower alkylcarbamoyl groups or by one, two or three halogen atoms (except trihydroxy);

— a saturated or unsaturated aza-, thia-, oxa-, thiaza-, oxaza- or diazacyclic radical which may be fused to a phenyl nucleus and contains from 2 to 7 carbon atoms in the heterocyclic ring, and which may be substituted by one, two or more lower alkyl, hydroxy, lower alkoxy, nitro, carboxy, lower alkoxycarbonyl or lower alkanoyloxy groups;

— a heterocyclic-lower alkyl radical the heterocycle of which is a saturated or unsaturated aza-, thia-, oxa-, thiaza-, oxaza- or diazacyclic radical; a radical of an alkanecarboxylic acid containing at least 3 carbon atoms, lower alkenecarboxylic acid, a lower alkanedicarboxylic acid or a lower alkenedicarboxylic acid; a lower cycloalkanecarboxylic acid which is unsubstituted or substituted by one or more hydroxy, lower alkoxy, carboxy, lower alkoxycarbonyl, lower alkyl, lower alkanoyl or oxo groups or halogen atoms;

— a benzoyl or naphthoyl radical which may be substituted by hydroxy, lower alkoxy, lower alkanoyloxy, carboxy, lower alkoxycarbonyl, nitro or amino groups or by halogen atoms;

— a phenyl- or naphthyl-lower alkanoyl radical which is unsubstituted or substituted in the aromatic nucleus by hydroxy, lower alkoxy, carboxy, lower alkoxycarbonyl, lower alkanoyl, lower alkanoyloxy, nitro, amino or mono- or di-lower alkylamino or by halogen;

— a radical of an unsaturated aza-, thia-, oxa-, thiaza-, oxaza- or diaza-cyclic acid which may be fused to a phenyl radical; a lower alkoxycarbonyl radical; a phenoxycarbonyl radical; a carbamoyl radical which may be substituted by one or two lower alkyl phenyl or benzyl radicals which may be substituted in the ring by hydroxy, lower alkoxy groups or by halogen atoms;

— a radical of a lower alkylsulphonic acid or phenylsulfonic acid which may be substituted in the ring by one or more lower alkyl or lower alkoxy groups or by halogen atoms; or

— a radical of a phosphonic acid which is unsubstituted or esterified by one or two lower alkyl radicals or by one or two phenyl radicals, the term "lower" signifying a radical containing 1 to 7 carbon atoms, or a salt thereof.

2. (+) 3-O-Methyl-cyanidan-3-ol.
3. (+) 3-O-Butyl-cyanidan-3-ol.
4. (+) 3-O-Butyryl-cyanidan-3-ol.
5. (+) 3-O-(3,3-Dimethylbutanoyl)-cyanidan-3-ol.
6. (+) 3-O-(3-Carboxypropionyl)-cyanidan-3-ol.
7. (+) 3-O-Decanoyl-cyanidan-3-ol.
8. (+) 3-O-Dodecyl-cyanidan-3-ol.
9. (+) 3-O-Heptyl-cyanidan-3-ol.
10. (+) 3-O-Octadecyl-cyanidan-3-ol.
11. (+) 3-O-Palmitoyl-cyanidan-3-ol.
12. (+) 3-O-(2-Carboxycyclohexanecarbonyl)-cyanidan-3-ol.
13. (+) 3-O-Benzoyl-cyanidan-3-ol.
14. (+) 3-O-(4-Fluorobenzoyl-cyanidan-3-ol.
15. (+) 3-O-Protocatechyl-cyanidan-3-ol.
16. (+) 3-O-(Acetylsalicylyl)-cyanidan-3-ol.
17. (+) 3-O-(2-Carboxybenzoyl)-cyanidan-3-ol.
18. (+) 3-O-(N-Phenylcarbamoyl)-cyanidan-3-ol.
19. (+) 3-O-Methanesulphonyl-cyanidan-3-ol.
20. (+) 3-O-(4-Methylbenzenesulphonyl)-cyanidan-3-ol.
21. (+) 3-O-(2-Cyanoethyl)-cyanidan-3-ol.
22. (+) 3-O-(3-Aminopropyl)-cyanidan-3-ol.
23. (+) 3-O-Methoxymethyl-cyanidan-3-ol.
24. (+) 3-O-Ethoxycarbonyl-cyanidan-3-ol.

25. A pharmaceutical composition which contains a compound according to claim 1, or a pharmaceutically acceptable salt thereof, together with at least one pharmaceutical carrier.

26. A pharmaceutical composition which contains a compound according to any one of claims 2 to 24, or a pharmaceutically acceptable salt thereof, together with at least one pharmaceutical carrier.

27. A method of preparing a medicament for the treatment of diseases of the liver, which comprises the use of a compound according to claim 1.

28. A method of preparing a medicament for the treatment of diseases of the liver, which comprises the use of a compound according to any one of claims 2 to 24.

29. A process for the preparation of an O-substituted derivative of (+)-cyanidan-3-ol of the formula I

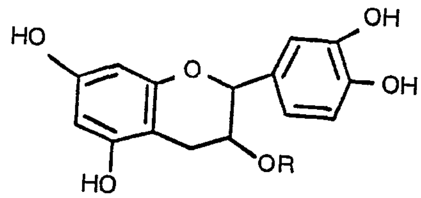

(I)

wherein R is as defined in claim 1, or of a salt thereof, which process comprises cleaving the radical $OR_1$ to the hydroxy group in a compound of the formula II

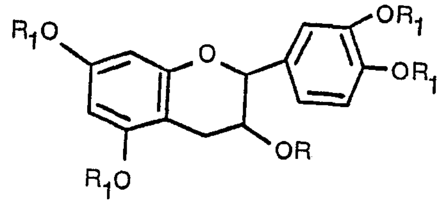

(II)

wherein $R_1O$ is a protected oxy group which is readily hydrolysable or hydrogenatable, and R is as defined above, and, if desired, converting a radical R in a resultant compound by reduction, and/or converting a resultant salt into the free compound or a free compound containing salt-forming groups into the salt.

**Revendications**

1. Nouveaux dérivés O-substitués du (+)-cyanidan-3-ol de formule I

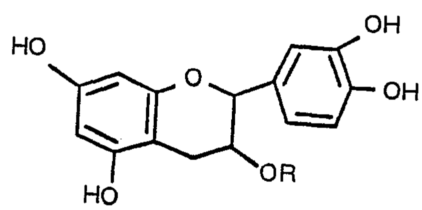

(I)

où R représente

un radical alcoyle ou alcényle non substitué ou substitué par un hydroxy, un alcanoyloxy inférieur, un alcoyloxy inférieur, un alcanoyle inférieur, un cyano, un amino, un mono- ou di-alcoyle inférieur-amino, un alcoylène inférieur-amino, un oxa- ou aza-alcoylène inférieur-amino, un carbamoyle, un mono- ou di-alcoyle inférieur-carbamoyle ou des atomes d'halogène,

un radical cycloalcoyle ayant de 3 à 6 atomes de carbone dans son noyau, non substitué ou susbtitué par un ou plusieurs hydroxy, alcoxy inférieur, alcoyle inférieur, carboxy, oxo, alcanoyle inférieur, amino, mono- ou di-alcoyl-amino ou carboalcoxy inférieur,

un radical cycloalcoyl-alcoyle inférieur, qui présente dans son noyau de 3 à 6 atomes de carbone et qui peut être non substitué ou substitué par au moins un radical alcoyle inférieur,

un radical phényle ounaphtyle, qui peut être mono-, di- ou trisubstitué par un hydroxy, un alcoxy inférieur, un carboxy, un carboalcoxy inférieur, un nitro, un amino, un mono- ou di-alcoyle inférieur-amino, ou des atomes d'halogène,

un radical phényl- ou naphtyl-alcoyle inférieur ou -alcényle inférieur qui peut être mono-, di- ou tri-substitué dans son noyau aromatique par un hydroxy, un alcoxy inférieur, un carboxy, un carbo-alcoxy inférieur, un alcanoyle, un nitro, un amino, un mono- ou dialcoyl-amino, un di-alcoyle inférieur-carbamoyle ou des atomes d'halogène (à l'exception du trihydroxy),

un radical aza-, thia-, oxa-, thiaza-, oxaza- ou diaza-cyclique saturé ou insaturé, qui peut être condensé sur un noyau phényle, et qui présente de 2 à 7 atomes de carbone dans le noyau hétérocyclique et qui peut être mono-, di- ou polysubstitué par un alcoyle, un hydroxy, un alcoxy inférieur, un nitro, un carboxy, un carbo-alcoxy inférieur ou un alcanoyloxy,

un radical hétérocyclyl-alcoyle inférieur, dont l'hétérocycle représente un cycle aza, thia, oxa, thiaza, oxaza ou diaza saturé ou insaturé,

un radical d'un acide alcane-carboxylique ayant au moins 3 atomes de carbone, d'un acide alcène inférieur-carboxylique, d'un acide alcane inférieur- dicarboxylique, ou d'un acide alcène inférieur-dicarboxylique,

un radical acide cycloalcane-carboxylique non substitué ou substitué par un ou plusieurs hydroxy, alcoyloxy inférieur, carboxy, carboalcoxy inférieur, alcoyle inférieur, alcanoyle inférieur, oxo ou halogène,

un radical benzoyle ou naphtoyle, qui peut le cas échéant être substitué par un hydroxy, un alcoxy inférieur, un alcanoyloxy, un carboxy, un carboalcoxy inférieur, un nitro, un amino ou un halogène,

un radical phényl- ou naphtyl-alcanoyle inférieur, qui peut être dans sa fraction aromatique non substitué ou substitué par un hydroxy, un alcoxy inférieur, un carboxy, un carboalcoxy inférieur, un alcanoyle inférieur, un alcanoyloxy inférieur, un nitro, un amino, un mono- ou un di-alcoyle inférieur-amino ou un halogène,

un radical d'un acide aza-, thia-, oxa-, thiaza-, oxaza ou diaza-cyclique, qui peut être saturé ou insaturé, ou encore condensé sur un radical phényle,

un radical alcoxycarbonyle,

un radical phényloxycarbonyle,

un radical carbamoyle, qui le cas échéant peut être mono- ou disubstitué par des radicaux alcoyle inférieur, des radicaux phényle ou benzyle, qui le cas échéant peuvent être substitués dans leur noyau par un hydroxy, un alcoxy inférieur, un alcoyloxy inférieur, ou des atomes d'halogène,

un radical d'un acide alcoyle inférieur-sulfonique, ou d'un acide phénylsulfonique, qui peut être substitué dans son noyau par un alcoyle inférieur, un alcoyloxy inférieur ou des atomes d'halogène, ou

le radical d'un acide phosphorique non substitué ou estérifié par 1 ou 2 radicaux alcoyle inférieur ou 1 ou 2 radicaux phényle, l'expression inférieur désignant un radical ayant jusqu'à 7 atomes de carbone, et leurs sels.

2. 3-O-méthyl-(+)-cyanidan-3-ol.
3. 3-O-butyl-(+)-cyanidan-3-ol.
4. 3-O-butyryl-(+)-cyanidan-3-ol.
5. 3-O-(3,3-diméthyl-butanoyl)-(+)-cyanidan-3-ol.
6. 3-O-(3-carboxy-propionyl)-(+)-cyanidan-3-ol.
7. 3-O-décanoyl-(+)-cyanidan-3-ol.
8. 3-O-dodécyl-(+)-cyanidan-3-ol.
9. 3-O-heptyl-(+)-cyanidan-3-ol.
10. 3-O-octadécyl-(+)-cyanidan-3-ol.
11. 3-O-palmitoyl-(+)-cyanidan-3-ol.
12. 3-O-(2-carboxy-cyclohexan-carbonyl)-(+)-cyanidan-3-ol.
13. 3-O-benzoyl-(+)-cyanidan-3-ol.
14. 3-O-(4-fluoro-benzoyl)-(+)-cyanidan-3-ol.
15. 3-O-protocatéchyl-(+)-cyanidan-3-ol.
16. 3-O-acétylsalicylyl-(+)-cyanidan-3-ol.
17. 3-O-(2-carboxy-benzoyl)-(+)-cyanidan-3-ol.
18. 3-O-(N-phénylcarbamoyl)-(+)-cyanidan-3-ol.
19. 3-O-méthanesulfonyl-(+)-cyanidan-3-ol.
20. 3-O-(4-méthyl-benzènesulfonyl)-(+)-cyanidan-3-ol.
21. 3-O-(2-cyano-éthyl)-(+)-cyanidan-3-ol.
22. 3-O-(3-amino-propyl)-(+)-cyanidan-3-ol.
23. 3-O-méthoxyméthyl-(+)-cyanidan-3-ol.
24. 3-O-éthoxycarbonyl-(+)-cyanidan-3-ol.
25. Compositions pharmaceutiques caractérisées en ce qu'elles contiennent un composé selon la revendication 1 ou un de ses sels pharmaceutiquement acceptables et au moins un support pharmaceutique.
26. Compositions pharmaceutiques caractérisées en ce qu'elles contiennent un composé selon l'une des revendications 2—24 ou un de ses sels pharmaceutiquement acceptables, et au moins un support pharmaceutique.
27. Application d'un composé selon la revendication 1 comme substance active pour préparer un médicament pour le traitement des maladies hépatiques.
28. Application d'un composé selon l'une des revendications 2—24 comme substance active pour préparer un médicament pour le traitement des maladies hépatiques.
29. Procédé de préparation de dérivés O-substitués du (+)-cyanidan-3-ol de formule I

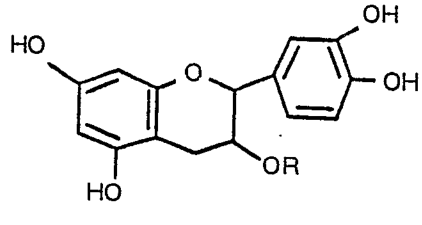

(I)

où R a la signification donnée dans la revendication 1, et de leurs sels, caractérisé en ce que dans un composé de formule II

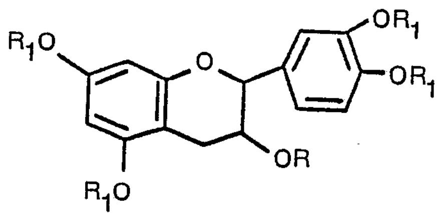

(II)

où $R_1O$ représente un groupe oxy protégé, qui est facilement hydrolysable ou hydrogénolysable, et R a la signification donnée ci-dessus, on clive la radical $OR_1$ pour donner un groupe hydroxy et, si on le désire, dans un composé obtenu on transforme un radical R par réduction, et/ou en ce que on transforme un sel obtenu en le composé libre ou un composé libre ayant des groupes salificateurs en le sel.